(19) Europäisches Patentamt
European Patent Office
Office européen des brevets

(11) Publication number : **0 471 516 A1**

# (12) EUROPEAN PATENT APPLICATION

(21) Application number : **91307341.7**

(22) Date of filing : **09.08.91**

(51) Int. Cl.[5] : **C07D 219/06**, C07D 219/08, C07D 471/04, C07D 491/04, A61K 31/435

(30) Priority : **16.08.90 GB 9018044**

(43) Date of publication of application : **19.02.92 Bulletin 92/08**

(84) Designated Contracting States : **AT BE CH DE DK ES FR GB GR IT LI LU NL SE**

(71) Applicant : **IMPERIAL CHEMICAL INDUSTRIES PLC**
**Imperial Chemical House, Millbank**
**London SW1P 3JF (GB)**

(72) Inventor : **Butlin, Roger John**
**38 Gawsworth Road**
**Macclesfield, Cheshire SK11 8BU (GB)**
Inventor : **Glarvey, Dickson**
**22 Swaledale Avenue**
**Congleton, Cheshire CW12 2BY (GB)**

(74) Representative : **Slatcher, Reginald Peter et al**
**Imperial Chemical Industries PLC Legal Department: Patents PO Box 6**
**Welwyn Garden City Herts, AL7 1HD (GB)**

## (54) Tricyclic heterocyclic derivatives.

(57) The invention relates to tricyclic heterocyclic derivatives, or pharmaceutically-acceptable salts or in vivo hydrolysable esters thereof, which possess anti-cancer activity. The invention also relates to processes for the manufacture of said tricyclic heterocyclic derivatives, to novel pharmaceutical compositions containing them and to the use of said tricyclic heterocyclic derivatives in the manufacture of a medicament for the production of an anti-cancer effect.

The invention provides an optionally substituted tricyclic heterocyclic derivative of the formula I

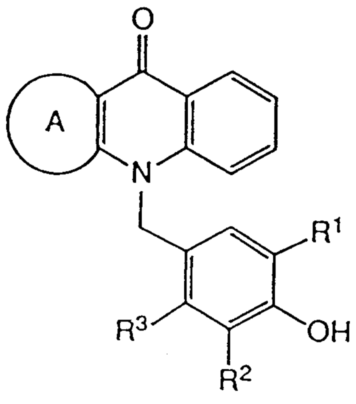

I

wherein A together with the adjacent vinylene group of the 4-oxo-1,4-dihydropyrid-1-yl ring completes a benzene or pyridine ring ;

$R^1$ and $R^2$, which may be the same or different, each is (1-4C)alkyl or (1-4C)alkoxy ;

and $R^3$ is hydrogen, (1-4C)alkyl or (1-4C)alkoxy ;

or a pharmaceutically-acceptable salt or in vivo hydrolysable ester thereof.



Jouve, 18, rue Saint-Denis, 75001 PARIS

This invention relates to novel tricyclic heterocyclic derivatives and more particularly it relates to tricyclic heterocyclic derivatives, or pharmaceutically-acceptable salts or in vivo hydrolysable esters thereof, which possess anti-cancer activity by virtue of an interaction with one or more of the enzymes known as the topoisomerases. The invention also relates to processes for the manufacture of said tricyclic heterocyclic derivatives and to novel pharmaceutical compositions containing them. The invention also relates to the use of a topoisomerase-affecting amount of said tricyclic heterocyclic derivatives in the production of an anti-cancer effect in the human or animal body and to the use of said tricyclic heterocyclic derivatives in the manufacture of a medicament for such use.

The group of enzymes known as the topoisomerases, including topoisomerase-I and II, are involved in the breaking and reforming of single or double strands of DNA. This may be required for example to change the topology of portions of DNA during transcription and replication in processes such as catenation and knotting which are essential for cell survival [Annual Reviews Biochem. 1981, 50, 879 and 1985, 54, 665].

Certain known anti-cancer agents are believed to act by interaction with topoisomerase enzymes. Thus, for example, anti-cancer drugs such as doxorubicin, amsacrine, ellipticine and etoposide have topoisomerase-II-affecting activity [J. Biol. Chem., 1984a, 259, 9182; Cancer Res., 1986, 46, 2021]. It is believed that damage is done to the DNA of the cancer cells by drug-induced stabilisation of a topoisomerase-DNA complex during a necessary breaking of the DNA chain. Thereby reformation of the DNA chain is inhibited. The result is a damaging breakage in the DNA chain which ultimately leads to cell death.

Anti-cancer drugs such as etoposide have been shown to give promising results in the treatment of various cancers in humans such as small-cell lung cancer, testicular cancer, lymphomas and leukaemias (New England J. Med., 1985, 312, 692). It is in addition expected that such anti-cancer drugs will in due course be shown to be effective against other cancers such as cancer of the colon, bladder, breast, ovary, liver, prostate, thyroid and stomach, and other cancers of the lung in addition to small-cell lung cancer.

The anti-cancer activity of compounds such as etoposide may be assessed in vitro by determining the DNA strand breakages which result from their interaction with topoisomerase-II, in cell culture by their inhibitory effect on cancer cell lines such as the small-cell lung cancer cell line N417A and the mouse leukaemia cell line L1210, and in vivo in mice by inhibition of the growth of tumours such as N417A and L1210 xenografts arising from the injection of N417A small-cell lung cancer cells or L1210 mouse leukaemia cells.

Other anti-cancer compounds may therefore be identified and compared with the known human anti-cancer compound etoposide by their ability to interact with topoisomerase-II and their activity in vitro and/or in vivo against the small-cell lung cancer cell line N417A and/or the mouse leukaemia cell line L1210.

We have now found that the tricyclic heterocyclic derivatives of the present invention interact with topoisomerase-II resulting in DNA strand breakages and inhibit the growth of the small-cell lung cancer cell line N417A and the leukaemia cell line L1210 and hence are of value as anti-cancer agents in warm-blooded animals such as man.

Certain tricyclic heterocyclic derivatives having structures related to the structures of the tricyclic heterocyclic derivatives of the present invention are known but it is believed that the tricyclic heterocyclic derivatives of the present invention are novel. Thus certain 10-alkyl-9,10-dihydroacridin-9-one derivatives which are stated to possess antiviral activity are disclosed in US Patent No. 4244954;

various 1-amino-10-alkyl-4-nitro-9,10-dihydroacridin-9-one derivatives which are stated to possess anti-tumour activity are disclosed in European Patent Application No. 0145226; and a 10-aryl-5,10-dihydropyrido[2,3-b][1,6]naphthyridin-5-one is disclosed in US Patent No. 4810708.

According to the invention there is provided a tricyclic heterocyclic derivative of the formula I (set out hereinafter) wherein A together with the adjacent vinylene group of the 4-oxo-1,4-dihydropyrid-1-yl ring completes a benzene or pyridine ring, which may optionally bear one, two or three substituents selected from halogeno, hydroxy, amino, (1-4C)alkyl, (2-4C)alkenyl, (2-C)alkynyl, (1-4C)alkoxy, (1-4C)alkylamino, di-[(1-4C-)alkyl]amino, piperidino, morpholino, piperazin-1-yl, 4-[(1-4C)alkyl]piperazin-1-yl, (1-6C)alkylthio, (1-6C)alkylsulphinyl, (1-6C)alkylsulphonyl, (2-4C)alkenylthio, (2-4C)alkenylsulphinyl, (2-4C)alkenylsulphonyl, (1-4C)alkylenedioxy, hydroxy-(1-4C)alkyl, hydroxy-(2-4C)alkylthio, hydroxy-(2-4C)alkylsulphinyl and hydroxy-(2-4C)alkylsulphonyl, from a group of the formula:-

$$-X^1-(CH_2)_m-N(R^4)-R^5$$

wherein $X^1$ is oxy, thio, sulphinyl, sulphonyl or imino, m is an integer from 2 to 4, and $R^4$ and $R^5$, which may be the same or different, each is hydrogen or (1-4C)alkyl, or $R^4$ and $R^5$ together with the nitrogen atom to which each is attached define a piperidino, morpholino, piperazin-1-yl or 4-[(1-4C)alkyl]piperazin-1-yl ring, or $X^1$ is a direct link, m is an integer from 1 to 4 and $R^4$ and $R^5$ have any of the meanings defined above,

and from phenyl-(1-4C)alkyl, phenyl-(1-4C)alkoxy, phenyl-(1-4C)alkylthio, phenyl-(1-4C)alkylsulphinyl and phenyl-(1-4C)alkylsulphonyl, and wherein any of said substituents comprising a phenyl group may optionally bear a halogeno, (1-4C)alkyl or (1-4C)alkoxy substituent;

$R^1$ and $R^2$, which may be the same or different, each is (1-4C)alkyl or (1-4C)alkoxy;
and $R^3$ is hydrogen, (1-4C)alkyl or (1-4C)alkoxy;
or a pharmaceutically-acceptable salt or in vivo hydrolysable ester thereof.

The chemical formulae referred to herein by Roman numerals are set out for convenience on a separate sheet hereinafter.

In this specification the generic term "alkyl" includes both straight-chain and branched-chain alkyl groups. However references to individual alkyl groups such as "propyl" are specific for the straight-chain version only and references to individual branched-chain alkyl groups such as "isopropyl" are specific for the branched-chain version only. An analogous convention applies to other generic terms.

Suitable values for the generic terms referred to above include those set out below.

A suitable value for the tricyclic heterocyclic radical defined by the 4-oxo-1,4-dihydroquinolin-1-yl ring and A is, for example, 9-oxo-9,10-dihydroacridin-10-yl, 5-oxo-5,10-dihydrobenzo[b][1,8]naphthyridin-10-yl, 5-oxo-5,10-dihydrobenzo[b][1,7]naphthyridin-10-yl, 10-oxo-5,10-dihydrobenzo[b][1,6]naphthyridin-5-yl or 10-oxo-5,10-dihydrobenzo[b][1,5]naphthyridin-5-yl.

Suitable values for the substituents which may be present on the tricyclic heterocyclic radical, or where appropriate for a substituent on a substituent comprising a phenyl group, include, for example:-

| | |
|---|---|
| for halogeno: | fluoro, chloro and bromo; |
| for (1-4C)alkyl: | methyl, ethyl, propyl, isopropyl, butyl and isobutyl; |
| for (2-4C)alkenyl: | vinyl and allyl; |
| for (2-4C)alkynyl: | ethynyl and 2-propynyl; |
| for (1-4C)alkoxy: | methoxy, ethoxy, propoxy, isopropoxy and butoxy; |
| for (1-4C)alkylamino: | methylamino, ethylamino, propylamino and isopropylamino; |
| for di-[(1-4C)alkyl]amino: | dimethylamino and diethylamino; |
| for 4-[(1-4C)alkyl]piperazin-1-yl: | 4-methylpiperazin-1-yl and 4-ethylpiperazin-1-yl; |
| for (1-6C)alkylthio: | methylthio, ethylthio, propylthio, isopropylthio, butylthio, isobutylthio, pentylthio and isopentylthio; |
| for (1-6C)alkylsulphinyl: | methylsulphinyl, ethylsulphinyl, propylsulphinyl, isopropylsulphinyl, butylsulphinyl, isobutylsulphinyl, pentylsulphinyl and isopentylsulphinyl; |

| | |
|---|---|
| for (1-6C)alkylsulphonyl: | methylsulphonyl, ethylsulphonyl, propylsulphonyl, isopropylsulphonyl, butylsulphonyl, isobutylsulphonyl, pentylsulphonyl and isopentylsulphonyl; |
| for (2-4C)alkenylthio: | vinylthio and allylthio; |
| for (2-4C)alkenylsulphinyl: | vinylsulphinyl and allylsulphinyl; |
| for (2-4C)alkenylsulphonyl: | vinylsulphonyl and allylsulphonyl; |
| for (1-4C)alkylenedioxy: | methylenedioxy and ethylenedioxy; |
| for hydroxy-(1-4C)alkyl: | hydroxymethyl, 2-hydroxyethyl and 3-hydroxypropyl; |
| for hydroxy-(2-4C)alkylthio: | 2-hydroxyethylthio and 3-hydroxypropylthio; |
| for hydroxy-(2-4C)-alkylsulphinyl: | 2-hydroxyethylsulphinyl and 3-hydroxypropylsulphinyl; |
| for hydroxy-(2-4C)alkyl-sulphonyl: | 2-hydroxyethylsulphonyl and 3-hydroxypropylsulphonyl; |
| for phenyl-(1-4C)alkyl: | benzyl, phenethyl and 3-phenylpropyl; |
| for phenyl-(1-4C)alkoxy: | benzyloxy, 2-phenylethoxy and 3-phenylpropoxy; |
| for phenyl-(1-4C)alkylthio: | benzylthio, 2-phenylethylthio and 3-phenylpropylthio; |
| for phenyl-(1-4C)alkyl-sulphinyl: | benzylsulphinyl, 2-phenylethyl-sulphinyl and 3-phenylpropyl-sulphinyl; |
| for phenyl-(1-4C)alkyl-sulphonyl: | benzylsulphonyl, 2-phenylethyl-sulphonyl and 3-phenylpropyl-sulphonyl. |

When a substituent on the tricyclic heterocyclic radical is a group of the formula:-

$$-X^1-(CH_2)_m-N(R^4)-R^5$$

then a suitable value for $R^4$ or $R^5$, which may be the same or different, when each is (1-4C)alkyl is, for example, methyl, ethyl, propyl, isopropyl or butyl; and when $R^4$ and $R^5$ together with the nitrogen atom to which each is attached defines a 4-[(1-4C)alkyl]piperazin-1-yl ring then a suitable value for said (1-4C)alkyl group is, for example, methyl, ethyl or propyl.

A suitable value for $R^1$, $R^2$ or $R^3$ when each is (1-4C)alkyl is, for example, methyl, ethyl, propyl or isopropyl; and when each is (1-4C)alkoxy is, for example, methoxy, ethoxy, propoxy or isopropoxy.

A suitable pharmaceutically-acceptable salt of a tricyclic heterocyclic derivative of the invention is, for

example, an acid-addition salt of a tricyclic heterocyclic derivative of the invention which is sufficiently basic, for example an acid-addition salt with an inorganic or organic acid such as hydrochloric, hydrobromic, sulphuric, trifluoroacetic, citric or maleic acid; or, for example, a salt of a tricyclic heterocyclic derivative of the invention which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

A suitable in vivo hydrolysable ester of a tricyclic heterocyclic derivative of the invention, i.e. a pharmaceutically-acceptable ester which is hydrolysed in the human or animal body to produce the parent compound, is, for example, an ester formed by the reaction of a phenolic hydroxy group in the tricyclic heterocyclic derivative of the formula I with a suitable pharmaceutically-acceptable acid. Suitable in vivo hydrolysable ester groups include, for example, (1-6C)alkanoyloxy such as acetoxy, propionyloxy and pivaloyloxy, benzoyloxy, (1-4C)alkoxycarbonyloxy such as ethoxycarbonyloxy, N-(1-4C)alkylcarbamoyloxy such as N-ethylcarbamoyloxy, or N,N-di[(1-4C)alkyl]carbamoyloxy such as N,N-dimethylcarbamoyloxy. Further suitable in vivo hydrolysable esters may be formed by the reaction of a phenolic hydroxy group in the tricyclic heterocyclic derivative of the formula I with a suitable pharmaceutically-acceptable inorganic acid such as sulphuric or phosphoric acid, or an alkali or alkaline earth metal or ammonium salt thereof such as a mono- or, where appropriate, a di-cationic salt, for example a sodium, calcium, magnesium or ammonium salt. Such in vivo hydrolysable esters may be identified by administering an ester to a test animal, for example by the conventional oral, subcutaneous or intravenous routes and subsequently by determining the quantity of the parent phenolic compound in the test animal's body fluids such as in its blood.

Particular novel compounds of the invention are, for example, tricyclic heterocyclic derivatives of the formula I wherein:-

(a) the tricyclic heterocyclic radical defined by the 4-oxo-1,4-dihydroquinolin-1-yl ring and A is 9-oxo-9,10-dihydroacridin-10-yl, 5-oxo-5,10-dihydrobenzo[b][1,8]naphthyridin-10-yl, 5-oxo-5,10-dihydrobenzo[b][1,7]naphthyridin-10-yl, 10-oxo-5,10-dihydrobenzo[b][1,6]naphthyridin-5-yl or 10-oxo-5,10-dihydrobenzo[b][1,5]-naphthyridin-5-yl; and the optional substituents on the tricyclic heterocycle, $R^1$, $R^2$ and $R^3$ have any of the meanings defined hereinbefore;

(b) the tricyclic heterocyclic radical defined by the 4-oxo-1,4-dihydroquinolin-1-yl ring and A is 9-oxo-9,10-dihydroacridin-10-yl or 5-oxo-5,10-dihydrobenzo[b][1,8]naphthyridin-10-yl; and the optional substituents on the tricyclic heterocycle, $R^1$, $R^2$ and $R^3$ have any of the meanings defined hereinbefore;

(c) the tricyclic heterocyclic radical bears one or two optional substituents selected from those defined hereinbefore; and the tricyclic heterocyclic radical, $R^1$, $R^2$ and $R^3$ have any of the meanings defined hereinbefore; or

(d) $R^1$ and $R^2$, which may be the same or different, each is methoxy or ethoxy, and $R^3$ is hydrogen; and the tricyclic heterocyclic radical and the optional substituents thereon have any of the meanings defined hereinbefore;

or a pharmaceutically-acceptable salt or in vivo hydrolysable ester thereof.

A particular compound of the invention comprises a tricyclic heterocyclic derivative of the formula I wherein A together with the adjacent vinylene group of the 4-oxo-1,4-dihydropyrid-1-yl ring completes a benzene or pyridine ring, which may optionally bear one, two or three substituents selected from halogeno, hydroxy, amino, (1-4C)alkyl, (1-4C)alkoxy, (1-4C)alkylamino, di-[(1-4C)alkyl]amino, piperidino, morpholino, piperazin-1-yl, 4-[(1-4C)alkyl]piperazin-1-yl, (1-6C)alkylthio, (1-6C)alkylsulphinyl, (1-6C)alkylsulphonyl and (1-4C)alkylenedioxy,

from a group of the formula:-

$$-X^1-(CH_2)_m-N(R^4)-R^5$$

wherein $X^1$ is oxy, thio, sulphinyl, sulphonyl or imino, m is an integer from 2 to 4, and $R^4$ and $R^5$, which may be the same or different, each is hydrogen or (1-4C)alkyl, or $R^4$ and $R^5$ together with the nitrogen atom to which each is attached define a piperidino, morpholino, piperazin-1-yl or 4-[(1-4C)alkyl]piperazin-1-yl ring, and from phenyl-(1-4C)alkyl, phenyl-(1-4C)alkoxy, phenyl-(1-4C)alkylthio, phenyl-(1-4C)alkylsulphinyl and phenyl-(1-4C)alkylsulphonyl, and wherein any of said substituents comprising a phenyl group may optionally bear a halogeno, (1-4C)alkyl or (1-4C)alkoxy substituent;

$R^1$ and $R^2$, which may be the same or different, each is (1-4C)alkyl or (1-4C)alkoxy;

and $R^3$ is hydrogen, (1-4C)alkyl or (1-4C)alkoxy;

or a pharmaceutically-acceptable salt or in vivo hydrolysable ester thereof.

A particular compound of the invention comprises a tricyclic heterocyclic derivative of the formula I wherein A together with the adjacent vinylene group of the 4-oxo-1,4-dihydroquinolin-1-yl ring completes a benzene or pyridine ring, which may optionally bear one, two or three substituents selected from fluoro, chloro, bromo, hydroxy, amino, methyl, ethyl, vinyl, ethynyl, methoxy, ethoxy, methylamino, ethylamino, dimethylamino, piperidino, morpholino, piperazin-1-yl, 4-methylpiperazin-1-yl, methylthio, ethylthio, propylthio, isopropylthio, isobutylthio, isopentylthio, methylsulphinyl, ethylsulphinyl, methylsulphonyl, ethylsulphonyl, vinylthio, vinylsulphinyl, vinylsulphonyl, methylenedioxy, 2-hydroxyethyl, 2-hydroxyethylthio, 2-hydroxyethylsulphinyl and 2-hydroxyethylsulphonyl,
from a group of the formula:-

$$-X^1-(CH_2)_m-N(R^4)-R^5.$$

wherein $X^1$ is oxy, thio, sulphinyl, sulphonyl or imino, m is 2 or 3, and $R^4$ and $R^5$, which may be the same or different, each is hydrogen, methyl or ethyl, or $R^4$ and $R^5$ together with the nitrogen atom to which each is attached define a piperidino, morpholino, piperazin-1-yl or 4-methylpiperazin-1-yl ring, or $X^1$ is a direct link, m is an integer from 1 to 3 and $R^4$ and $R^5$ have any of the meanings defined above, and from benzyl, phenethyl, benzyloxy and benzylthio and wherein any of said substituents comprising a phenyl group may optionally bear a substituent selected from fluoro, chloro, methyl and methoxy; $R^1$ and $R^2$, which may be the same or different, each is methyl, ethyl, methoxy or ethoxy;
and $R^3$ is hydrogen, methyl, ethyl, methoxy or ethoxy;
or a pharmaceutically-acceptable salt or in vivo hydrolysable ester thereof.

A further particular compound of the invention comprises a tricyclic heterocyclic derivative of the formula I wherein A together with the adjacent vinylene group of the 4-oxo-1,4-dihydroquinolin-1-yl ring completes a benzene or pyridine ring; the tricyclic heterocyclic radical so defined may optionally bear one or two substituents selected from fluoro, chloro, bromo, hydroxy, amino, methyl, ethyl, methoxy, ethoxy, methylamino, ethylamino, dimethylamino, piperidino, morpholino, piperazin-1-yl, 4-methylpiperazin-1-yl, methylthio, ethylthio, propylthio, isopropylthio, isobutylthio, isopentylthio, methylsulphinyl, ethylsulphinyl, methylsulphonyl, ethylsulphonyl and methylenedioxy, from a group of the formula:-

$$-X^1-(CH_2)_m-N(R^4)-R^5$$

wherein $X^1$ is oxy, thio or imino, m is 2, and $R^4$ and $R^5$, which may be the same or different, each is hydrogen, methyl or ethyl, or $R^4$ and $R^5$ together with the nitrogen atom to which each is attached define a piperidino, morpholino, piperazin-1-yl or 4-methylpiperazin-1-yl ring, and from benzyl, phenethyl, benzyloxy and benzylthio and wherein any of said substituents comprising a phenyl group may optionally bear a substituent selected from fluoro, chloro, methyl and methoxy;
$R^1$ and $R^2$, which may be the same or different, each is methyl, ethyl, methoxy or ethoxy;
and $R^3$ is hydrogen, methyl, ethyl, methoxy or ethoxy;
or a pharmaceutically-acceptable salt or in vivo hydrolysable ester thereof.

A preferred compound of the invention comprises a tricyclic heterocyclic derivative of the formula I wherein the tricyclic heterocyclic radical defined by the 4-oxo-1,4-dihydroquinolin-1-yl ring and A is 9-oxo-9,10-dihydroacridin-10-yl or 5-oxo-5,10-dihydrobenzo[b][1,8]naphthyridin-10-yl, which may optionally bear one or two substituents selected from fluoro, chloro, hydroxy, methoxy, ethoxy, methylthio, ethylthio, isopentylthio, methylsulphinyl, ethylsulphinyl, methylsulphonyl, ethylsulphonyl, methylenedioxy, 2-dimethylaminoethoxy, 2-dimethylaminoethylthio, 2-dimethylaminoethylamino and benzylthio;
$R^1$ is methyl, ethyl, methoxy or ethoxy;
$R^2$ is methyl, ethyl, methoxy or ethoxy; and
$R^3$ is hydrogen;
or a pharmaceutically-acceptable salt or in vivo hydrolysable ester thereof.

A further preferred compound of the invention comprises a tricyclic heterocyclic derivative of the formula I wherein the tricyclic heterocyclic radical defined by the 4-oxo-1,4-dihydroquinolin-1-yl ring and A is 9-oxo-9,10-dihydroacridin-10-yl or 5-oxo-5,10-dihydrobenzo[b][1,8]naphthyridin-10-yl, which may optionally bear one or two substituents selected from fluoro, chloro, bromo, hydroxy, amino, ethynyl, methoxy, ethoxy, piperidino, morpholino, piperazin-1-yl, 4-methylpiperazin-1-yl, methylthio, ethylthio, isopentylthio, methylsulphinyl, ethylsulphinyl, methylsulphonyl, ethylsulphonyl, vinylthio, vinylsulphinyl, vinylsulphonyl, methylenedioxy, 2-hydroxyethylthio, 2-aminoethoxy, 2-dimethylaminoethoxy, 2-diethylaminoethoxy, 2-aminoethylthio, 2-dimethylaminoethylthio, 2-diethylaminoethylthio, 2-aminoethylsulphinyl, 2-dimethylaminoethylsulphinyl, 2-diethylaminoethylsulphinyl, 2-aminoethylsulphonyl, 2-dimethylaminoethylsulphonyl, 2-diethylaminoethylsulphonyl, 2-dimethylaminoethylamino and benzylthio;
$R^1$ is methoxy;
$R^2$ is methoxy; and
$R^3$ is hydrogen;
or a pharmaceutically-acceptable salt or in vivo hydrolysable ester thereof.

An especially preferred compound of the invention comprises a tricyclic heterocyclic derivative of the formula I wherein the tricyclic heterocyclic radical defined by the 4-oxo-1,4-dihydroquinolin-1-yl ring and A is 9-oxo-9,10-dihydroacridin-10-yl or 5-oxo-5,10-dihydrobenzo[b][1,8]naphthyridin-10-yl, which may optionally bear

one substituent selected from chloro, methylthio, methylenedioxy and 2-dimethylaminoethylthio;
$R^1$ is methoxy;
$R^2$ is methoxy; and
$R^3$ is hydrogen;
or a pharmaceutically-acceptable salt thereof.

A further especially preferred compound of the invention comprises a tricyclic heterocyclic derivative of the formula I wherein the tricyclic heterocyclic radical defined by the 4-oxo-1,4-dihydroquinolin-1-yl ring and A is 9-oxo-9,10-dihydroacridin-10-yl which bears one or two substituents selected from fluoro, chloro, hydroxy, methoxy, methylthio, methylsulphinyl, methylsulphonyl, methylenedioxy, 2-aminoethoxy, 2-aminoethylthio, 2-dimethylaminoethylthio, 2-aminoethylsulphonyl and 2-dimethylaminoethylsulphonyl;
$R^1$ is methoxy;
$R^2$ is methoxy; and
$R^3$ is hydrogen;
or a pharmaceutically-acceptable salt thereof.

A further especially preferred compound of the invention comprises a tricyclic heterocyclic derivative of the formula I wherein the tricyclic heterocyclic radical defined by the 4-oxo-1,4-dihydroquinolin-1-yl ring and A is 9-oxo-9,10-dihydroacridin-10-yl which bears a substituent in the 3-position selected from fluoro, chloro, hydroxy, methoxy, methylthio, 2-dimethylaminoethylthio, 2-aminoethoxy, 2-aminoethylsulphonyl and 2-dimethylaminoethylsulphonyl, or a 2,3-methylenedioxy substituent;
$R^1$ is methoxy;
$R^2$ is methoxy; and
$R^3$ is hydrogen;
or a pharmaceutically-acceptable salt thereof.

Specific especially preferred compounds of the invention include, for example, the following tricyclic heterocyclic derivatives of the formula I, or pharmaceutically-acceptable salts thereof:-
3-chloro-10-(4-hydroxy-3,5-dimethoxybenzyl)-9,10-dihydroacridin-9-one, 10-(4-hydroxy-3,5-dimethoxybenzyl)-2,3-methylenedioxy-9,10-dihydroacridin-9-one,
10-(4-hydroxy-3,5-dimethoxybenzyl)-3-methylthio-9,10-dihydroacridin-9-one,
3-(2-dimethylaminoethylthio)-10-(4-hydroxy-3,5-dimethoxybenzyl)-9,10-dihydroacridin-9-one,
10-(4-hydroxy-3,5-dimethoxybenzyl)-5,10-dihydrobenzo[b][1,8]naphthyridin-5-one,
3-fluoro-10-(4-hydroxy-3,5-dimethoxybenzyl)-9,10-dihydroacridin-9-one,
3-chloro-2-fluoro-10-(4-hydroxy-3,5-dimethoxybenzyl)-9,10-dihydroacridin-9-one,
3-hydroxy-10-(4-hydroxy-3,5-dimethoxybenzyl)-9,10-dihydroacridin-9-one,
10-(4-hydroxy-3,5-dimethoxybenzyl)-3-(piperazin-1-yl)-9,10-dihydroacridin-9-one,
3-(2-dimethylaminoethylsulphonyl)-10-(4-hydroxy-3,5-dimethoxybenzyl)-9,10-dihydroacridin-9-one,
3-(2-aminoethoxy)-10-(4-hydroxy-3,5-dimethoxybenzyl)-9,10-dihydroacridin-9-one and
3-(2-aminoethylsulphonyl)-10-(4-hydroxy-3,5-dimethoxybenzyl)-9,10-dihydroacridin-9-one.

A compound of the invention comprising a tricyclic heterocyclic derivative of the formula I, or a pharmaceutically-acceptable salt or in vivo hydrolysable ester thereof, may be prepared by any process known to be applicable to the preparation of structurally-related compounds. Such procedures are provided as a further feature of the invention and are illustrated by the following representative examples in which, unless otherwise stated, A, $R^1$, $R^2$, $R^3$, $X^1$, m, $R^4$ and $R^5$ have any of the meanings defined hereinbefore.

(a) The coupling of a compound of the formula II with a compound of the formula III wherein Z is a displaceable group;
provided that the phenolic hydroxy group in the compound of the formula III and any amino, alkylamino, piperazinyl or other hydroxy group in the compounds of the formulae II and III may be protected by a conventional protecting group or alternatively any such group need not be protected, whereafter any such protecting group is removed by conventional means.

A suitable displaceable group Z is, for example, a halogeno, sulphonyl or sulphonyloxy group such as chloro, bromo, iodo, mesyl, methanesulphonyloxy and toluene-p-sulphonyloxy.

The coupling reaction is preferably carried out in the presence of a suitable base such as, for example, an alkali or alkaline earth metal carbonate, (1-4C)alkoxide, hydroxide or hydride, for example sodium carbonate, potassium carbonate, sodium ethoxide, potassium butoxide, sodium hydroxide, potassium hydroxide, sodium hydride or potassium hydride, or, for example, an organic amine base such as, for example, pyridine, lutidine, collidine, 4-dimethylaminopyridine, triethylamine, morpholine or diazabicyclo[5.4.0]undec-7-ene.

The coupling reaction is conveniently performed in a suitable inert solvent or diluent, for example N,N-dimethylformamide, N,N-dimethylacetamide, dimethylsulphoxide, acetone, 1,2-dimethoxyethane or tetrahydrofuran, and at a temperature in the range, for example -10 to 150°C, conveniently at or near ambient

temperature.

Alternatively the coupling reaction may be performed utilising a phase transfer catalyst, for example a tetra-(1-4C)alkylammonium salt such as tetrabutylammonium hydroxide or hydrogen sulphate, a suitable alkali or alkaline earth metal hydroxide as defined above and a suitable inert solvent or diluent, for example methylene chloride or methyl ethyl ketone, and at a temperature in the range, for example 10 to 140°C, conveniently at or near 70°C.

A suitable protecting group for the phenolic hydroxy group in the compound of the formula III is, for example, an arylmethyl group (especially benzyl), a tri-(1-4C)alkylsilyl group (especially trimethylsilyl or tert-butyldimethylsilyl), an aryldi-(1-4C)alkylsilyl group (especially dimethylphenylsilyl), a diaryl-(1-4C)alkylsilyl group (especially tert-butyldiphenylsilyl), a (1-4C)alkyl group (especially methyl and isopropyl), a (2-4C)alkenyl group (especially allyl), a (1-4C)alkoxymethyl group (especially methoxymethyl) or a tetrahydropyranyl group (especially tetrahydropyran-2-yl). The deprotection conditions for the above protecting groups will necessarily vary with the choice of protecting group. Thus, for example, an arylmethyl group such as a benzyl group may be removed, for example, by hydrogenation over a catalyst such as palladium-on-charcoal. Alternatively a trialkylsilyl or an aryldialkylsilyl group such as a tert-butyldimethylsilyl or a dimethylphenylsilyl group may be removed, for example, by treatment with a suitable acid such as hydrochloric, sulphuric, phosphoric or trifluoroacetic acid, or with an alkali metal or ammonium fluoride such as sodium fluoride or, preferably, tetrabutylammonium fluoride. Alternatively an alkyl group may be removed, for example, by treatment with an alkali metal (1-4C)alkylsulphide such as sodium thioethoxide or, for example, by treatment with an alkali metal diarylphosphide such as lithium diphenylphosphide or, for example, by treatment with a boron or aluminium trihalide such as boron tribromide. Alternatively a (2-4C)alkenyl group may be removed, for example, by treatment with an organometallic catalyst such as an organopalladium catalyst, for example tetrakis(triphenylphosphine)palladium(0), in the presence of a carbon nucleophile such as the carbanion formed from a weak acid such as Meldrum's acid. Alternatively a (1-4C)alkoxymethyl group or tetrahydropyranyl group may be removed, for example, by treatment with a suitable acid such as hydrochlotic or trifluoroacetic acid.

A suitable protecting group for an amino, alkylamino or piperazinyl group is, for example, an acyl group, for example a (2-4C)alkanoyl group (especially acetyl), a (1-4C)alkoxycarbonyl group (especially methoxycarbonyl, ethoxycarbonyl or tert-butoxycarbonyl), an arylmethoxycarbonyl group (especially benzyloxycarbonyl) or an aroyl group (especially benzoyl). The deprotection conditions for the above protecting groups necessarily vary with the choice of protecting group. Thus, for example, an acyl group such as an alkanoyl or alkoxycarbonyl or an aroyl group may be removed for example, by hydrolysis with a suitable base such as an alkali metal hydroxide, for example lithium or sodium hydroxide. Alternatively an acyl group such as a tert-butoxycarbonyl group may be removed, for example, by treatment with a suitable acid such as hydrochloric, sulphuric or phosphoric acid or trifluoroacetic acid, and an arylmethoxycarbonyl group such as a benzyloxycarbonyl group may be removed, for example, by hydrogenation over a catalyst such as palladium-on-charcoal.

A suitable protecting group for a hydroxy group is, for example, an acyl group, for example a (2-4C)alkanoyl group (especially acetyl), an aroyl group (especially benzoyl) or an arylmethyl group (especially benzyl). The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as benzyl group may be removed, for example, by hydrogenation over a catalyst such as palladium-on-charcoal.

The starting materials of the formulae II and III may be obtained by standard procedures of organic chemistry. The preparation of such starting materials is described, for the purpose of illustration only, in the accompanying non-limiting Examples. Alternatively necessary starting materials are obtainable by analogous procedures or by modifications thereto which are within the ordinary skill of an organic chemist.

(b) The cyclisation of a compound of the formula IV wherein Z is a displaceable group as defined hereinbefore.

The cyclisation reaction is preferably carried out in the presence of a suitable base such as, for example, an alkali or alkaline earth metal (1-4C)alkoxide, hydroxide or hydride, for example sodium ethoxide, potassium butoxide, sodium hydroxide, potassium hydroxide, sodium hydride or potassium hydride. The cyclisation reaction is conveniently performed in a suitable inert solvent or diluent, for example N,N-dimethylformamide, N,N-dimethylacetamide, dimethylsulphoxide, acetone, 1,2-dimethoxyethane or tetrahydrofuran, and at a temperature in the range, for example -10 to 150°C, conveniently at or near ambient temperature.

The starting material of the formula IV may be obtained by standard procedures of organic chemistry. The preparation of such starting materials is described, for the purpose of illustration only, in the accompanying non-limiting Examples. Alternatively necessary starting materials are obtainable by analogous procedures or by modifications thereto which are within the ordinary skill of an organic chemist.

(c) For the production of a compound of the formula I wherein the tricyclic heterocyclic radical bears a hydroxy or substituted hydroxy substituent, an amino or substituted amino substituent or a substituted mercapto substituent, the displacement reaction of a compound of the formula I which bears a displaceable substituent Z as defined hereinbefore with an appropriate alcohol, amine or thiol.

The displacement reaction is preferably carried out in the presence of a suitable base as defined hereinbefore and in a suitable inert solvent or diluent, for example, N,N-dimethylformamide, N,N-dimethylacetamide, dimethylsulphoxide, N-methylpyrrolidin-2-one, acetone, tetrahydrofuran or ethanol, and at a temperature in the range, for example, 10 to 150°C, conveniently at or near ambient temperature.

(d) For the production of a compound of the formula I wherein the tricyclic heterocyclic radical bears a substituent comprising a sulphinyl or sulphonyl group, the oxidation of a compound of the formula I wherein the tricyclic heterocyclic radical bears an appropriate substituent comprising a thio group.

A suitable oxidising agent is, for example, any agent known in the art for the oxidation of thio to sulphinyl and/or sulphonyl, for example, hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible and with the required stoichiometric amount of oxidising agent in order to reduce the risk of over oxidation and damage to other functional groups. In general the reaction is carried out in a suitable solvent or diluent such as methylene chloride, chloroform, acetone, tetrahydrofuran or tert-butyl methyl ether and at a temperature, for example, at or near ambient temperature, that is in the range 15 to 35°C. When a compound carrying a sulphinyl group is required a milder oxidising agent may also be used, for example sodium or potassium metaperiodate, conveniently in a polar solvent such as acetic acid or ethanol. It will be appreciated that when a compound of the formula I containing a sulphonyl group is required, it may be obtained by oxidation of the corresponding sulphinyl compound as well as of the corresponding thio compound.

(e) For the production of a compound of the formula I wherein the tricyclic heterocyclic radical bears a substituent of the formula:-

$$-X^1-(CH_2)_m-N(R^4)-R^5$$

and $X^1$ is oxy, thio or imino, the alkylation of a compound of the formula I wherein the tricyclic heterocyclic radical bears a substituent of the formula $-X^1-H$ with a compound of the formula:- $Z-(CH_2)_m-N(R^4)-R^5$ wherein Z is a displaceable group.

The alkylation reaction is preferably carried out in the presence of a suitable base as defined hereinbefore and in a suitable inert solvent or diluent such as N,N-dimethylformamide, N,N-dimethylacetamide, dimethylsulphoxide, N-methylpyrrolidin-2-one, acetone or tetrahydrofuran, and at a temperature in the range, for example, 10 to 150°C, conveniently at or near 60°C.

(f) For the production of a compound of the formula I wherein the tricyclic heterocyclic radical bears an amino substituent, the reduction of a compound of the formula I wherein the tricyclic heterocyclic radical bears a nitro substituent.

A suitable reducing agent is, for example, any agent known in the art for the reduction of a nitro group to an amino group. Thus, for example, the reduction may be carried out by the hydrogenation of a solution of the nitro compound in an inert solvent or diluent in the presence of a suitable metal catalyst, for example finely divided platinum metal (obtained by the reduction of platinum oxide in situ). A suitable inert solvent or diluent is, for example, an alcohol, for example methanol, ethanol or isopropanol, or an ether, for example tetrahydrofuran.

A further suitable reducing agent is, for example an activated metal such as activated tin which may be produced, for example, by treatment of a tin salt such as stannous chloride with a reducing agent such as an alkali metal or alkaline earth metal hydride, for example sodium borohydride. Thus for example, the reduction may be carried out by stirring a mixture of the nitro compound and the activated metal in a suitable solvent or diluent such as an alcohol, for example, methanol or ethanol at a temperature in the range, for example 10 to 150°C, conveniently at or near ambient temperature.

When a pharmaceutically-acceptable salt of a novel compound of the formula I is required, it may be obtained, for example, by reaction of said compound with a suitable acid or base using a conventional procedure.

When an in vivo hydrolysable ester of a novel compound of the formula I is required, it may be obtained, for example, by reaction of said compound with a suitable esterifying reagent using a conventional procedure.

Many of the intermediates of the formulae II and III are novel and these are provided as a further feature of the invention.

As stated hereinbefore a tricyclic heterocyclic derivative of the formula I is capable of interaction with the enzyme topoisomerase-II and hence is of value as an anti-cancer agent in the human or animal body which is in need of such treatment. Both in-vitro and in vivo screening methods for anti-cancer agents utilising human

or animal cancer cell lines are well known to the man skilled in the art and are within the ordinary skill of the research team involved in the discovery of new anti-cancer agents. Examples of such screening methods have been disclosed as follows:- an in vivo test involving L1210 leukaemia (European Patent Application No. 0165592); and an in vivo test involving P388 murine leukaemia and in vitro tests involving several tumour cell lines (European Patent Application No. 0329108). The effects of the interaction of a tricyclic heterocyclic derivative of the formula I with topoisomerase-II may be demonstrated using one or more of the test procedures set out below:-

(a) An in vitro assay which determines the ability of a test compound to interact with the enzyme topoisomerase-II, obtained in partially purified form from human HeLa cervical carcinoma cells grown in cell culture. Aliquots of the topoisomerase-II so obtained are added to a mixture of plasmid DNA [0.5 ng of a linearised pUC18 plasmid labelled with [$^{32}$P]-ATP], test compound (dissolved in dimethylsulphoxide) and a conventional buffer solution comprising essential salts, bovine serum albumin, ATP, EDTA and 2-mercaptoethanol. The mixture is incubated at 30°C for 10 minutes. Sodium dodecylsulphate and proteinase K are added in turn to give final concentrations of 1% w/v and 2 mg/ml respectively and the mixture is heated to 45°C to effect proteolysis. The resultant mixture is analysed by electrophoresis on a polyacrylamide gel at pH 8.3 and 450 volts for one hour. Thereafter the gel is fixed, dried and autoradiogrammed to determine the extent of DNA fragmentation.

(b) Any assay which determines the ability of a test compound to inhibit the growth of the small-cell lung cancer cell line N417A in cell culture. The test is similar to that described by T. Mosmann (J. Immunol. Meths., 1983, 65, 55-63) but using the tetrazolium salt method of J. Carmichael et al. (J. Cancer Res., 1987, 47, 936-942) to assess the reductive capacity of any viable cells.

(c) An assay which determines the ability of a test compound to inhibit the growth of the mouse leukaemia cell line L1210 in cell culture. The test is similar to those described in UK Patent Specification No. 2065653B; and in J. Med. Chem., 1985, 28, 1468.

(d) An assay which determines the ability of a test compound to achieve an anti-cancer effect in vivo as shown by a delay in the growth of tumours induced in two groups of mice by the injection of N417A small-cell lung cancer cells. The growth rate of the tumours so induced is monitored in one of the groups of mice which serves as the control group. The other group of mice is dosed with the test compound. Comparison of the growth rates in the two groups of animals allows an anti-cancer effect to be demonstrated.

(e) An assay which determines the ability of a test compound to achieve an anti-cancer effect in vivo as shown by an increase in survival time of mice inoculated with L1210 mouse leukaemia cells.

Although the pharmacological properties of the compounds of the formula I vary with structural change as expected, in general activity possessed by compounds of the formula I may be demonstrated at the following concentrations or doses in one or more of the above tests (a)-(e):-

Test (a) :- significant activity at approximatelyl100 μM which is equivalent to that given by etoposide at a concentration in the range, for example, 10-200 μM;

Test (b) :- $IC_{50}$ in the range, for example, 0.1-40 μM;

Test (c) :- $IC_{50}$ in the range, for example, 0.1-50 μM;

Test (d) :- Growth delay obtained at a dose in the range, for example, 1-200 mg/kg/day;

Test (e) :- Increased survival time at a dose in the range, for example, 1-200 mg/kg/day.

Thus, by way of example, the compound 3-chloro-10-(4-hydroxy-3,5-dimethoxybenzyl)-9,10-dihydroacridin-9-one possesses significant activity in Test (a) at 100 μM which is equivalent to that possessed by etoposide at 25 μM, and has an $IC_{50}$ of 0.4 μM against the N417A small-cell lung cancer cell line [Test (b)];
the compound 10-(4-hydroxy-3,5-dimethoxybenzyl)-5,10-dihydrobenzo[b][1,8]naphthyridin-5-one possesses significant activity in Test (a) at 100 μM which is equivalent to that possessed by etoposide at 50 μM and has an $IC_{50}$ of 3.7 μM in Test (b);
the compound 3-(2-dimethylaminoethylsulphonyl)-10-(4-hydroxy-3,5-dimethoxybenzyl)-9,10-dihydroacridin-9-one possesses significant activity in Test (a) at 100 μM which is equivalent to that possessed by etoposide at 100 μM, and has an $IC_{50}$ of 0.8 μM in Test (b).

According to a further feature of the invention there is provided a pharmaceutical composition which comprises a tricyclic heterocyclic derivative of the formula I, or a pharmaceutically-acceptable salt or in vivo hydrolysable ester thereof, in association with a pharmaceutically-acceptable diluent or carrier.

The composition may be in a form suitable for oral administration, for example, as a tablet or capsule; or especially for parenteral injection (including intravenous, subcutaneous, intramuscular, intravascular or infusion), as a sterile solution, suspension or emulsion; or for topical administration, for example, as an ointment or cream; or for rectal administration as a suppository.

In general the above compositions may be prepared in a conventional manner using conventional excipients.

The tricyclic heterocyclic derivative of the formula I will normally be administered to a warm-blooded animal at a unit dose within the range 50-5000 mg per square metre body area of the animal, i.e. approximately 1-100

mg/kg, and this normally provides a therapeutically-effective dose. A unit dose form such as a tablet or capsule will usually contain, for example, 1-250 mg of active ingredient. Preferably a dose in the range of 1-100 mg/kg is employed. However the daily dose will necessarily be varied depending upon the host treated, the particular route of administration, and the severity of the illness being treated. Accordingly the optimum dosage will be determined by the practitioner who is treating any particular patient.

The anti-cancer treatment defined hereinbefore may be applied as a sole therapy or may involve, in addition to the tricyclic heterocyclic derivative of the formula I, one or more other anti-cancer substances selected from, for example, mitotic inhibitors, for example vinblastine; alkylating agents, for example cis-platin, carboplatin and cyclophosphamide; antimetabolites, for example 5-fluorouracil, cytosine arabinoside and hydroxyurea; thymidylate synthase inhibitors such as those described in UK Patent No. 2188319B; intercalating antibiotics, for example adriamycin and bleomycin; enzymes, for example asparaginase; other topoisomerase inhibitors, for example etoposide and biological response modifiers, for example interferon. Such conjoint treatment may be achieved by way of the simultaneous, sequential or separate dosing of the individual components of the treatment. According to this aspect of the invention there is provided a pharmaceutical product comprising a tricyclic heterocyclic derivative of the formula I, or a pharmaceutically-acceptable salt or in vivo hydrolysable ester thereof, and an additional anti-tumour substance as defined hereinbefore for the conjoint treatment of cancer.

According to a further feature of the invention there is provided a tricyclic heterocyclic derivative of the formula I, or a pharmaceutically-acceptable salt or in vivo hydrolysable ester thereof, for use in a method of treatment of the human or animal body by therapy.

According to a further feature of the invention there is provided the use of a tricyclic heterocyclic derivative of the formula I, or a pharmaceutically-acceptable salt or in vivo hydrolysable ester thereof, in the manufacture of a medicament for use in the production of an anti-cancer effect in the human or animal body.

According to a further feature of the invention there is provided a method for producing an anti-cancer effect in the human or animal body in need of such treatment which comprises administering to said body a topoisomerase-affecting amount of a tricyclic heterocyclic derivative of the formula I, or a pharmaceutically-acceptable salt or in vivo hydrolysable ester thereof.

A tricyclic heterocyclic derivative of the formula I is of value against a wide range of cancers. The anti-cancer agent etoposide is known to possess topoisomerase-II-affecting activity and to give promising results in the treatment of various cancers in humans such as small-cell lung cancer, testicular cancer, lymphomas and leukaemias. Consequently it is reasonably expected that a tricyclic heterocyclic derivative of the formula I will possess anti-cancer activity against these cancers. In addition it is expected that, as cancerous tissue necessarily requires functioning topoisomerases for continued growth, a compound which possesses the ability to induce DNA strand breakages will be effective against other cancers such as cancer of the colon, bladder, breast, ovary, liver, prostate, thyroid and stomach, and other cancers of the lung in addition to small-cell lung cancer.

The invention will now be illustrated by the following non-limiting Examples in which, unless otherwise stated:-

(i) evaporations were carried out by rotary evaporation in vacuo and work-up procedures were carried out after removal of residual solids by filtration;

(ii) operations were carried out at room temperature, that is in the range 18-25°C and under an atmosphere of an inert gas such as argon;

(iii) column chromatography (by the flash procedure) and medium pressure liquid chrmatography (MPLC) were performed on Merck Kieselgel silica (Art. 9385) or Merck Lichroprep RP-18 (Art. 9303) reversed-phase silica obtained from E. Meck, Darmstadt, W. Germany;

(iv) yields are given for illustration only and are not necessarily the maximum attainable;

(v) the end-products of the formula I have satisfactory microanalyses and their structures were confirmed by NMR and mass spectral techniques;

(vi) intermediates were not generally fully characterised and purity was assessed by thin layer chrmatographic, infra-red (IR) or NMR analysis;

(vii) melting points are uncorrected and were determined using a Mettler SP62 automatic melting point apparatus or an oil-bath apparatus; melting points for the end-products of the formula I were determined after recrystallisation from a conventional organic solvent such as ethanol, methanol, acetone, ether or hexane, alone or in admixture; and

(viii) the following abbreviations have been used:-

| | |
|---|---|
| THF | tetrahydrofuran; |
| DMS0 | dimethylsulphoxide; |
| DM | N,N-dimethylformamide; |
| DMA | N,N-dimethylacetamide. |

## EXAMPLE 1

Sodium hydride (60% w/w dispersion in mineral oil, 0.876g) was added to a suspension of 9-acridone (4.145g) in DMF (40ml) which was cooled in a cold water bath, and the mixture was stirred for 45 minutes. 4-Tert-butyldimethylsilyloxy-3,5-dimethoxybenzyl chloride (7g) was added and the mixture was stirred at ambient temperature for 20 hours. The mixture was poured onto water and extracted with ethyl acetate. The organic phase was washed with water, dried ($MgSO_4$) and evaporated. The residue was purified by column chromatography using a 3:1 v/v mixture of hexane and ethyl acetate as eluent. There was thus obtained 10-(4-tert-butyldimethylsilyloxy-3,5-dimethoxybenzyl)-9,10-dihydroacridin-9-one (7.86g, 78%) as a pale yellow solid, m.p. 209-210°C.

NMR ($CDCl_3$): 0.1(s, 6H), 1.0(s, 9H), 3.6(s, 6H), 5.5(s, 2H), 6.4(s, 2H), 7.3(t, 2H), 7.4(d, 2H), 7.6(m, 2H), 8.6(m, 2H).

A portion (2.30g) of the material so obtained was dissolved in THF (100ml), the solution was cooled to -70°C and tetrabutylammonium fluoride (1M in THF; 4.5ml) was added. The mixture was allowed to warm to -25°C over a period of 1 hour and maintained at this temperature for 30 minutes. The reaction mixture was poured onto a saturated aqueous ammonium chloride solution and extracted three times with ethyl acetate. The organic layers were washed twice with water and once with brine, dried ($MgSO_4$) and evaporated. The residue was purified by column chromatography using increasingly polar mixtures of hexane and ethyl acetate as eluent. There was thus obtained 10-(4-hydroxy-3,5-dimethoxybenzyl)-9,10-dihydroacridin-9-one (1.38g, 62%) as a yellow solid, m.p. 217°C (decomp.).

NMR ($d_6$-DMSO): 3.7(s, 6H), 5.75(s, 2H), 6.55(s, 2H), 7.4(m, 2H), 7.8(s, 2H), 7.85(m, 2H), 8.4(s, 0.8H), 8.4(broad s, 1H), 8.45(m, 2H).

Elemental Analysis: Found C, 60.2; H, 4.0, N, 2.9;

$C_{22}H_{19}NO_4$. 0.8 $CHCl_3$ requires C, 59.9; H, 4.4; N, 3.1%.

The starting material, 4-tert-butyldimethylsilyloxy-3,5-dimethoxybenzyl chloride, was prepared as follows:-

Imidazole (9.11g) and tert-butyldimethylsilyl chloride (9.11g) were added in turn to a solution of 3,5-dimethoxy-4-hydroxybenzaldehyde (10.5g) in DMF (25ml) and the mixture was stirred at ambient temperature for 6 hours. A further portion of tert-butyldimethylsilyl chloride (0.3g) was added and the mixture was stirred for 20 hours. The reaction mixture was partitioned between water and diethyl ether. The organic extracts were washed with water and brine, dried ($MgSO_4$) and evaporated to give crude 4-tert-butyldimethylsilyloxy-3,5-dimethoxybenzaldehyde (17.5g) as a solid. The product so obtained was dissolved in methanol (150ml) and sodium borohydride (1.3g) was added over a 20 minute period, the solution having been cooled to 0°C. The mixture was stirred for a further 20 minutes at 0°C. The solvent was removed by evaporation and the residue was treated with 5% w/v aqueous acetic acid. The resulting solution was extracted with diethyl ether. The organic phase was dried ($MgSO_4$) and evaporated to give 4-tert-butyldimethylsilyloxy-3,5-dimethoxybenzyl alcohol (17.2g).

NMR ($CDCl_3$): 0.1(s, 6H), 1.0(s, 9H), 1.7(s, 1H), 3.8(s, 6H), 4.6(s, 2H), 6.5(s, 2H).

To a solution of 4-tert-butyldimethylsilyloxy-3,5-dimethoxybenzyl alcohol (17.2g) in dichloromethane (220ml) which had been cooled to -10°C was added a solution of thionyl chloride (4.6ml) in dichloromethane (10ml). The reaction mixture was allowed to warm to 0°C over a period of 20 minutes. The mixture was poured into aqueous sodium hydrogen carbonate and extracted with dichloromethane. The organic extracts were washed with water, dried ($MgSO_4$) and evaporated to give the required starting material (18.0g, 99% yield) as a solid, m.p. 38-39°C.

NMR ($CDCl_3$): 0.1(s, 6H), 1.0(s, 9H), 3.8(s, 6H), 4.5(s, 2H), 6.5(s, 2H).

## EXAMPLE 2

In a manner similar to that described in Example 1 the compounds disclosed in Table I were prepared. The yields given represent the total yield over the two step process.

TABLE I

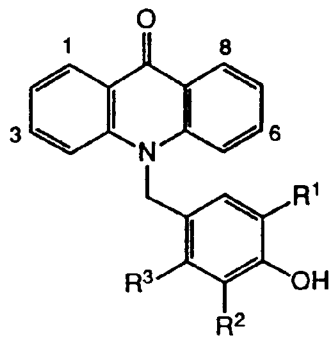

| Ex.2 Compound No. | Substituent | $R^1$ | $R^2$ | $R^3$ | m.p. (°C) | Yield (%) |
|---|---|---|---|---|---|---|
| 1[a] | 3-chloro | OMe | OMe | H | 223-225 | 30 |
| 2[b] | 1-chloro | OMe | OMe | H | 215 decomp. | 2 |
| 3[c] | 2,3-methylenedioxy | OMe | OMe | H | 248 decomp. | 8 |
| 4[d,e] | 1-fluoro | OMe | OMe | H | 205 decomp. | 41 |
| 5[f] | 1-fluoro-6,7-methylenedioxy | OMe | OMe | H | 300 decomp. | 3 |
| 6[g] | 2-fluoro | OMe | OMe | H | 230 decomp. | 3 |
| 7[h] | - | Me | Me | H | 253 decomp. | 1 |

## Notes

a) The required starting material, 3-chloro-9,10-dihydroacridin-9-one, was prepared as follows:-
A mixture of 2,4-dichlorobenzoic acid (19.1g), potassium carbonate (14g), copper bronze (1g) and an excess of aniline (100ml) was heated to reflux for 2.5 hours. The excess of aniline was removed by steam distillation to give 2-anilino-4-chlorobenzoic acid (12.3g, 50%), m.p. 198-202°C. The material so obtained was heated on a steam bath with phosphorous oxychloride (100ml) for 3 hours after which the mixture was evaporated to yield 3,9-dichloroacridine. To this crude product was added 2N aqueous hydrochloric acid and the mixture was heated to reflux for 3 hours. The solvent was removed by evaporation to yield the

required starting material (8.5g, 75%).
NMR ($d_6$-DMSO) : 7.2-7.3(m, 2H), 7.5-7.6(m, 2H), 7.7-7.8(m, 1H), 8.2-8.25(d, 2H).
b) The starting material, 1-chloro-9,10-dihydroacridin-9-one, is disclosed in US Patent No. 4,244,954.
c) The preparation of 2,3-methylenedioxy-9,10-dihydroacridin-9-one is described in Annalen, 1966, 691, 134.
d) The product was purified by recrystallisation from a mixture of methanol, dichloromethane and hexane.
e) The preparation of 1-fluoro-9,10-dihydroacridin-9-one is described in J. Chem. Soc., 1947, 759.
f) The product gave the following NMR data:- ($d_6$-DMSO) 3.6(s, 6H), 5.6(s, 2H), 6.1(s, 2H), 6.4(s, 2H), 7.0(m, 1H), 7.1(s, 1H), 7.4(d, 1H), 7.6-7.7(m, 2H).
The preparation of 1-fluoro-6,7-methylenedioxy-9,10-dihydroacridin-9-one was as follows:-
A mixture of 2-chloro-6-fluorobenzoic acid (3.02g), 3,4-methylenedioxyaniline (2.37g), potassium acetate (3.4g), triethylamine (2.41ml) and copper (II) acetate (0.1g) in isopropanol (100ml) was heated to reflux for 23 hours. Evaporation of the solvent gave a dark residue which was partitioned between 1N aqueous hydrochloric acid and ethyl acetate. Evaporation of the organic extract yielded 2-fluoro-6-(3,4-methylenedioxyanilino)benzoic acid (3.6g, 76%), as a brown solid.
A mixture of a portion (2.5g) of the acid so obtained and polyphosphoric acid (27g) was heated to 140°C for 1 hour. The mixture was poured into hot water (150ml), allowed to cool to 25°C and the pH was taken to pH5 by the addition of aqueous sodium hydroxide solution (15% w/v). The precipitate was filtered off, washed with water and dried. There was thus obtained 1-fluoro-6,7-methylenedioxy-9,10-dihydroacridin-9-one (0.78g, 33%) as a dark powdery solid.
NMR ($d_6$-DMSO): 6.2(s, 2H), 6.85(m, 1H), 6.9(s, 1H), 7.2(d, 1H), 7.4(s, 1H), 7.6(m, 1H).
g) The preparation of 2-fluoro-9,10-dihydroacridin-9-one is described in Liebigs Ann. Chem., 1976, 946.
h) The preparation of 4-hydroxy-3,5-dimethylbenzyl chloride was by direct chlorination of 4-hydroxy-3,5-dimethylbenzyl alcohol with thionyl chloride using the procedure described in the last paragraph of the portion of Example 1 which is concerned with the preparation of starting materials.

## EXAMPLE 3

The procedure described in Example 1 was repeated except that 5,10-dihydrobenzo[b][1,8]naphthyridin-5-one was used in place of 9-acridone and 3,4,5-trimethoxybenzyl chloride was used in place of 4-tert-butyldimethylsillyloxy-3,5-dimethoxybenzyl chloride. There was thus obtained 10-(3,4,5-trimethoxybenzyl)-5,10-dihydrobenzo[b][1,8]naphthyridin-5-one in 25% yield, m.p. 198-201°C.

To a solution of 10-(3,4,5-trimethoxybenzyl)-5,10-dihydrobenzo[b][1,8]naphthyridin-5-one (150mg) in dichloromethane (20ml) which had been cooled to 0°C was added boron trichloride (1.0M in dichloromethane; 4.5ml). The mixture was allowed to warm to ambient temperature and stirred for 24 hours. The mixture was quenched with a saturated aqueous sodium bicarbonate solution and extracted with ethyl acetate (3 x 25ml). The organic extracts were washed with water (2 x 25ml) and brine, dried ($MgSO_4$) and evaporated. The residue was recrystallised from a mixture of ethyl acetate and hexane. There was thus obtained 10-(4-hydroxy-3,4-dimethoxybenzyl)-5,10-dihydrobenzo[b][1,8]naphthyridin-5-one as a pale brown solid (32mg, 28%), m.p. 225-228°C.
NMR ($d_6$-DMSO): 3.59(s, 6H), 5.98(s, 2H), 6.48(s, 2H), 7.3-7.5(m, 2H), 7.75 (m, 2H), 8.3(broad s, 1H), 8.3(m, 1H), 8.7(m, 1H), 8.9(m, 1H).

The preparation of 5,10-dihydrobenzo[b][1,8]naphthyridin-5-one is described in J. Med. Chem., 1977, 20, 1242.

## EXAMPLE 4

A mixture of 3-chloro-9,10-dihydroacridin-9-one (230mg), 2-butanone (2ml), 50% w/v aqueous sodium hydroxide solution (2ml) and benzyltriethylammonium chloride (20mg) was stirred rapidly and heated to 70°C. 4-Tert-butyldimethylsilyloxy-3,5-dimethoxybenzyl chloride (320mg) was added and the mixture was heated to 70°C for 3 hours. The mixture was quenched with water and extracted with ethyl acetate (3 x 20ml). The combined extracts were dried ($MgSO_4$) and evaporated. The residue was purified by column chromatography using increasingly polar mixtures of hexane and ethyl acetate as eluent. The product so obtained was recrystallised from a mixture of acetone and hexane to give 3-chloro-10-(4-hydroxy-3,5-dimethoxybenzyl)-9,10-dihydroacridin-9-one (68mg, 17%) as a pale yellow solid, m.p. 223-225°C.
NMR ($CDCl_3$): 3.75(s, 6H), 5.47(s 2H), 5.5(s, 1H), 6.4(s, 2H), 7.25(m, 4H), 7.65(m, 1H), 8.52(d, 1H), 8.6(m, 1H);
Elemental Analysis: Found C, 65.2; H, 4.3; N, 3.2;

$C_{22}H_{18}ClNO_4$. 0.5 $H_2O$ requires C, 65.3; H, 4.7; N, 3.5%.

## EXAMPLE 5

The procedure described in Example 4 was repeated with the exception that the teactants were 6-chloro-2-methoxy-9,10-dihydroacridin-9-one and 3,4,5-trimethoxybenzyl chloride. There was thus obtained 6-chloro-2-methoxy-10-(3,4,5-trimethoxybenzyl)-9,10-dihydroacridin-9-one in 22% yield.

Demethylation of the 4-methoxy group was carried out using the procedure described in the second paragraph of Example 3 to yield 6-chloro-10-(4-hydroxy-3,5-dimethoxybenzyl)-2-methoxy-9,10-dihydroacridin-9-one in 63% yield, m.p. 300°C.

NMR ($d_6$-DMSO): 3.6(s, 6H), 3.9(s, 3H), 5.6(s, 2H), 6.4(s, 2H), 7.3(m, 1H), 7.4(m, 1H), 7.7(m, 3H), 8.4(m, 2H).

The acridinone starting material is described in Synthesis, 1982, 493.

## EXAMPLE 6

A solution of 3-chloro-10-(4-tert-butyldimethylsilyloxy-3,5-dimethoxybenzyl)-9,10-dihydroacridin-9-one (160mg) in 1-methylpyrrolidin-2-one (2ml) was stirred at ambient temperature and sodium methanethiolate (100mg) was added. The mixture was stirred at ambient temperature for 2 hours. The reaction mixture was quenched with water and extracted with ethyl acetate (3 x 25ml). The combined extracts were washed with water (3 x 25ml) and brine, dried ($MgSO_4$) and evaporated. The resulting crude product was purified by column chromatography using a 49:1 v/v mixture of dichloromethane and methanol as eluent. There was thus obtained 10-(4-hydroxy-3,5-dimethoxybenzyl)-3-methylthio-9,10-dihydroacridin-9-one (20mg, 15%), m.p. 204-207°C.

NMR ($CDCl_3$): 2.46(s, 3H), 3.77(s, 6H), 5.47(s, 3H), 6.42(s, 2H), 7.15(m, 2H), 7.3(m, 2H), 7.6(m, 1H), 8.84(d, 1H), 8.6(m, 1H).

## EXAMPLE 7

In a manner similar to that described in Example 6, except that sodium 2-dimethylaminoethanethiolate [prepared from sodium hydride and 2-dimethylaminoethanethiol in DMF (1ml)] was used in place of sodium methanethiolate, there was obtained 3-(2-dimethylaminoethylthio)-10-(4-hydroxy-3,5-dimethoxybenzyl)-9,10-dihydroacridin-9-one in 40% yield, m.p. 156-160°C.

NMR ($CDCl_3$): 2.6(s, 6H), 2.9(t, 2H), 3.4(t, 2H), 3.8(s, 6H), 5.65(s, 2H), 6.5(s, 2H), 7.2(m, 4H), 7.6(m, 1H), 8.4(d, 1H), 8.5(m, 1H).

## EXAMPLE 8

10-(4-Hydroxy-3,5-dimethoxybenzyl)-1-methylthio-9,10-dihydroacridin-9-one was prepared following a procedure similar to that described in Example 6, with the following exceptions:

1-chloro-10-(4-tert-butyldimethylsilyloxy-3,5-dimethoxybenzyl)-9,10-dihydroacridin-9-one was used in place of 3-chloro-10-(4-tert-butyldimethylsilyloxy-3,5-dimethoxybenzyl)-9,10-dihydroacridin-9-one; the solvent used was DMF; the reaction mixture was cooled initially to 0°C and then allowed to warm to ambient temperature; and, as the tert-butyldimethylsilyl group was not removed by these reaction conditions, the desilylation procedure described in the second paragraph of Example 1 was used.

The product was obtained in 11% yield as a solid, m.p. 206-209°C, and the following NMR data were obtained:- ($CDCl_3$): 2.5(s, 3H), 3.8(s, 6H), 5.45(s, 2H), 5.5(s, 1H), 6.4(s, 2H), 7.1(d, 2H), 7.3(m, 2H), 7.5(t, 1H), 7.6(m, 1H), 8.6(m, 1H).

## EXAMPLE 9

3-Chloro-10-(4-tert-butyldimethylsilyloxy-3,5-dimethoxybenzyl)-9,10-dihydroacridin-9-one (150mg) was added to a stirred suspension of sodium 3-methylbutanethiolate [prepared from sodium hydride (55mg of a 60% dispersion in mineral oil) and 3-methylbutanethiol (0.2ml) in DMF (2ml)] which had been cooled to 0°C. The reaction mixture was stirred at 0°C for 30 minutes. The mixture was partitioned between ethyl acetate (3 x 20ml) and water. The organic phase was washed with water (2 x 20ml) and brine, dried ($MgSO_4$) and evaporated to give 10-(4-tert-butyldimethylsilyloxy-3,5-dimethoxybenzyl)-3-isopentylthio-9,10-dihydroacridin-9-one which was used without further purification. The above product was desilylated using the procedure described in the second paragraph of Example 1. There was thus obtained 10-(4-hydroxy-3,5-dimethoxybenzyl)-3-isopentylthio-9,10-dihydroactidin-9-one as a yellow solid (76mg, 56%), m.p. 139-140°C.

NMR ($CDCl_3$): 0.8(d, 6H), 1.4(m, 2H), 1.6(m, 1H), 3.0(t, 2H), 3.4(s, 6H), 5.7(s, 2H), 6.5(s, 2H), 7.2(m, 1H), 7.3(m, 2H), 7.6(m, 1H), 7.7(m, 1H), 8.2(d, 1H), 8.4(m, 1H).

## EXAMPLE 10

In a manner similar to that described in Example 9 except that phenylmethanethiol was used in place of 3-methylbutanethiol, there was obtained 3-benzylthio-10-(4-hydroxy-3,5-dimethoxybenzyl)-9,10-dihydroacridin-9-one in 14% yield, m.p. 182-187°C.
NMR ($CDCl_3$): 3.7(s, 6H), 4.1(s, 2H), 5.3(s, 2H), 5.4(s, 1H), 6.3(s, 2H), 7.1-7.3(m, 9H), 7.5(m, 1H), 8.3(d, 1H), 8.5(m, 1H).

## EXAMPLE 11

In a manner similar to that described in Example 1 the compounds disclosed in Table II were prepared. The yields given represent the total yield over the two step process.

TABLE II

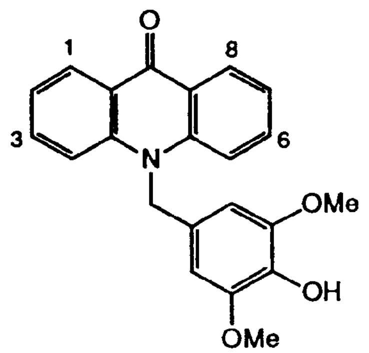

| Ex. 11 Compound No. | Substituent | m.p. (°C) | Yield (%) |
|---|---|---|---|
| 1 | 2-methoxy | 181-183 | 9 |
| 2[a] | 2-hydroxy | 215-218 | 17 |
| 3[b] | 3-bromo | oil | 5 |

Notes

a) The starting material, 2-tert-butyldimethylsilyloxy-9,10-dihydroacridin-9-one, was prepared as follows:-
A suspension of 2-methoxy-9,10-dihydroacridin-9-one (5.19g) in aqueous hydrobromic acid (48% w/v, 175ml) was heated to reflux for 12 hours. The mixture was cooled to ambient temperature and water (400ml) was added. The resultant precipitate was washed with water and dried in vacuo. There was thus obtained 2-hydroxy-9,10-dihydtoacridin-9-one (4.4g) which was used without further purification.

A mixture of a portion (0.377g) of the material so obtained, tert-butyldimethylsilyl chloride (0.330g), imidazole (0.303g) and DMF (5ml) was stirred at ambient temperature for 24 hours. The mixture was partitioned

between ethyl acetate and water. The organic phase was washed in turn with 2N aqeous hydrochloric acid, water and brine, dried ($MgSO_4$) and evaporated. There was thus obtained the required starting material (0.062g) which was used without further purification.

b) The product gave the following characteristic NMR data:- ($CD_3SOCD_3$) 3.48(s, 6H), 5.55(s, 2H), 6.35(s, 2H), 7.26(m, 1H), 7.36(d, 1H), 7.60(d, 1H), 7.69(m, 1H), 7.79(d, 1H), 8.17(d, 1H), 8.25(m, 1H).

The 3-bromo-9,10-dihydroacridin-9-one, used as a starting material, was obtained as follows:-

A mixture of 2-chloro-4-bromobenzoic acid (12.75g), aniline (30ml), potassium carbonate (8.5g) and copper bronze (0.3g) was heated to 140°C for 3 hours. The excess of aniline was removed by steam distillation. The residue was filtered and the filtrate was acidified by the addition of concentrated aqueous hydrochloric acid (10ml). The solid was isolated and dried to give 2-anilino-4-bromobenzoic acid (10.3g).

A mixture of a portion (8g) of the product so obtained and polyphosphoric acid (50g) was heated to 140°C for 3 hours. The mixture was poured onto a mixture of ice and dilute aqueous ammonium hydroxide solution. The precipitate so obtained was washed with a saturated sodium bicarbonate solution and with water and then dried. There was thus obtained 3-bromo-9,10-dihydroacridin-9-one (7.4g).

NMR:- ($CD_3SOCD_3$ + $CD_3CO_2D$) 7.2(t, 1H), 7.3(d, 1H), 7.5(d, 1H), 7.7(t, 2H), 8.1(d, 1H), 8.2(d, 1H).

## EXAMPLE 12

A solution of 10-(4-allyloxy-3,5-dimethoxybenzyl)-3-chloro-9,10-dihydroacridin-9-one (0.435g) in 1-methylpyrrolidin-2-one (10ml) was stirred at ambient temperature and sodium ethanethiolate (0.098g) was added portionwise. The mixture was stirred at ambient temperature for 1 hour. The mixture was partitioned between dichloromethane and water. The organic phase was washed with water and with brine, dried ($MgSO_4$) and evaporated. The residue was purified by column chromatography using initially dichloromethane and then increasingly polar mixtures of dichloromethane and ethyl acetate as eluent. There was thus obtained 10-(4-allyloxy-3,5-dimethoxybenzyl)-3-ethylthio-9,10-dihydroacridin-9-one (0.145g) as an oil.

A solution of tetrakis(triphenylphosphine)palladium(0) [0.04g] in THF (1ml) was added to a mixture of the product so obtained, 2,2-dimethyl-1,3-dioxane-4,6-dione (Meldrum's acid, 0.13g) and DMF (2ml). The mixture was stirred at ambient temperature for 2 hours. The mixture was partitioned between ethyl acetate and water. The organic phase was washed with water and with brine, dried ($MgSO_4$) and evaporated. The residue was purified by column chromatography using increasingly polar mixtures of dichloromethane and ethyl acetate as eluent. There was thus obtained 3-ethylthio-10-(4-hydroxy-3,5-dimethoxybenzyl)-9,10-dihydroacridin-9-one (0.07g, 52%), m.p. 195-198°C.

NMR ($CDCl_3$): 1.29(t, 3H), 2.93(q, 2H), 3.76(s, 6H), 5.46(s, 3H), 6.42(s, 2H), 7.15(m, 2H), 7.3(d, 1H), 7.38(d, 1H), 7.6(m, 1H), 8.4(d, 1H), 8.58(m, 1H).

The 10-(4-allyloxy-3,5-dimethoxybenzyl)-3-chloro-9,10-dihydroacridin-9-one used as a starting material was obtained as follows:-

A mixture of 3-chloro-9,10-dihydroacridin-9-one (0.516g), 2-butanone (2ml), 50% w/v aqueous sodium hydroxide (2ml), 4-allyloxy-3,5-dimethoxybenzyl chloride (0.6g) and benzyltriethylammonium chloride (0.03g) was stirred rapidly and heated to reflux for 3 hours. The mixture was cooled to ambient temperature and water (10ml) was added. The precipitated product was isolated and dried. There was thus obtained the required starting material (0.344g, 35%), m.p. 227°C (recrystallised from a mixture of hexane and dichloromethane).

The 4-allyl-3,5-dimethoxybenzyl chloride used above was obtained as follows:-

A mixture of 3,5-dimethoxy-4-hydroxybenzaldehyde (25g), allyl bromide (15ml), potassium carbonate (25g) and DMF (100ml) was stirred at ambient temperature for 16 hours. The mixture was partitioned between ethyl acetate and water. The organic phase was washed with water and with brine, dried ($MgSO_4$) and evaporated. There was thus obtained 4-allyloxy-3,5-dimethoxybenzaldehyde (33.8g).

The product so obtained was dissolved in methanol (200ml) and the solution was cooled to 0°C. Sodium borohydride (3.75g) was added portionwise and the mixture was stirred at 0°C for 30 minutes and at ambient temperature for 1 hour. The mixture was evaporated and the residue was partitioned between ethyl acetate and aqueous acetic acid (5% v/v). The organic phase was washed with water and with brine, dried ($MgSO_4$) and evaporated. There was thus obtained 4-allyloxy-3,5-dimethoxybenzyl alcohol (31.2g).

A solution of thionyl chloride (14ml) in dichloromethane (30ml) was added dropwise to a solution of the benzyl alcohol so obtained in dichloromethane (200ml) which had been cooled to -5°C. The mixture was stirred at -5°C for 1 hour. The mixture was poured into a mixture of ice and a saturated aqueous sodium bicarbonate solution and stirred for 30 minutes. The mixture was extracted with dichloromethane. The organic extracts were washed with water and with brine, dried ($MgSO_4$) and evaporated. There was thus obtained the required starting material (30g) as an oil.

## EXAMPLE 13

In a manner similar to that described in Example 12, the compounds disclosed in Table III were prepared. The appropriate sodium thiolate was prepared by the addition of the appropriate thiol to a stirred suspension of sodium hydride (60% w/w dispersion in mineral oil) in 1-methylpyrrolidin-2-one. The yields given represent the total yield over the two step process.

TABLE III

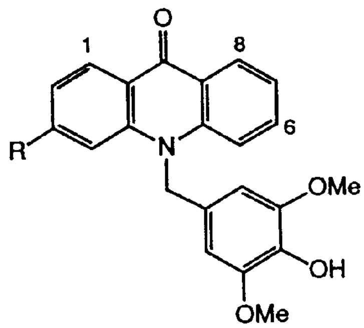

| Ex. 13 Compound No. | R | m.p. (°C) | Yield (%) |
|---|---|---|---|
| 1[a] | 2-hydroxyethylthio | 175-179 | 13 |
| 2 | 2-aminoethylthio | 149-152 | 35 |
| 3 | 2-diethylaminoethylthio | 152-154 | 25 |
| 4[b] | 2-dimethylaminoethoxy | 198-201 | 29 |
| 5[b] | 2-diethylaminoethoxy | 153-156 | 45 |
| 6[b] | 3-dimethylaminopropoxy | 145-146 | 49 |
| 7[b] | methoxy | 206-209 | 62 |
| 8[c] | hydroxy | 204-207 | 50 |
| 9[d] | 2-dimethylaminoethylamino | 159-162 | 25 |
| 10[d] | 4-methylpiperazin-1-yl | 199-202 | 28 |

Notes

a) The reaction mixture was heated to 100°C for 2 hours to complete the displacement reaction.

b) A mixture of equimolar amounts of the appropriate alcohol and potassium tert-butoxide was used in place of sodium ethanethiolate. The reaction mixture was heated to 85°C for 1 hour.

c) Allyl alcohol was used as the appropriate alcohol and the resultant 3-allyloxy-10-(4-allyloxy-3,5-dimethoxybenzyl)-9,10-dihydroacridin-9-one, on treatment with 2,2-dimethyl-1,3-dioxane-4,6-dione and tetrakis(triphenylphosphine)palladium(0), gave the required 3-hydroxy-9,10-dihydroacridin-9-one.

d) The appropriate amine was used in place of sodium ethanethiolate. The reaction mixture was heated to 140°C for 24 hours.

## EXAMPLE 14

Sodium hydride (80% w/v dispersion in mineral oil, 0.375g) was added to a solution of 2-[N-(4-allyloxy-3,5-dimethoxybenzyl)amino]2′,4-dichloro-5-fluorobenzophenone (0.375g) in DMF (8ml) and the mixture was stirred at ambient temperature for 30 minutes. Water (50ml) was added and the mixture was extracted with dichloromethane. The organic phase was washed with water, dried ($MgSO_4$) and evaporated. The residue was recrystallised from a mixture of hexane and dichloromethane. There was thus obtained 10-(4-allyloxy-3,5-dimethoxybenzyl)-3-chloro-2-fluoro-9,10-dihydroacridin-9-one (0.25g, 61%), m.p. 241-243°C.

The product so obtained was treated with 2,2-dimethyl-1,3-dioxane-4,6-dione and tetrakis(triphenylphosphine)palladium(0), using the procedure described in the second paragraph of Example 12. There was thus obtained 3-chloro-2-fluoro-10-(4-hydroxy-3,5-dimethoxybenzyl)-9,10-dihydroacridin-9-one in 20% yield, m.p. >250°C (decomposes).

The 2-[N-(4-allyloxy-3,5-dimethoxybenzyl)amino]-2′,4-dichloro-5-fluorobenzophenone used as a starting material was obtained as follows:-

A mixture of 4-chloro-5-fluoroanthranilic acid (1.55g; J. Het. Chem., 1986, 23, 1801), 4-allyloxy-3,5-dimethoxybenzyl chloride (1.98g), potassium carbonate (0.56g), potassium iodide (0.1g) and water (12ml) was stirred at ambient temperature for 90 minutes and then heated to reflux for 30 minutes. The mixture was cooled to ambient temperature. The precipitate was isolated, washed with water and dried. There was thus obtained 2-[N-(4-allyloxy-3,5-dimethoxybenzyl)-amino]-4-chloro-5-fluorobenzoic acid (1.8g, 55%), m.p. 176-179°C (recrystallised from a mixture of hexane and ethyl acetate).

A solution of di-(trichloromethyl) carbonate (1.29g) in toluene (15ml) was added to a vigorously stirred suspension of the product so obtained and sodium carbonate (0.65g) in water (15ml). The mixture was stirred at ambient temperature for 3 hours. The mixture was partitioned between ethyl acetate and water. The organic phase was washed with water and with brine, dried ($MgSO_4$) and evaporated. The residue was recrystallised from a mixture of hexane and ethyl acetate to give an orange solid (1.28g, 70%), m.p. 126-128°C.

n-Butyl lithium (1.6M in hexane, 1.09ml) was added dropwise to a solution of 2-chlorobromobenzene (0.205ml) in THF (100ml) which had been cooled to -75°C. A solution of a portion (0.736g) of the product obtained above in THF (2ml) was added and the mixture was stirred at -70°C for 2 hours and then allowed to warm to 0°C. The mixture was partitioned between ethyl acetate and a saturated aqueous ammonium chloride solution. The organic phase was washed with water and with brine, dried ($MgSO_4$) and evaporated. The residue was purified by column chromatography using a 6:1 v/v mixture of hexane and ethyl acetate as eluent. There was thus obtained 2-[N-(4-allyloxy-3,5-dimethoxybenzyl)amino]-2′,4-dichloro-5-fluorobenzophenone (0.628g, 73%), m.p. 121-123°C (recrystallised from a mixture of hexane and dichloromethane).

## EXAMPLE 15

Using a similar procedure to that described in Example 12, 10-(4-allyloxy-3,5-dimethoxybenzyl)-3-chloro-2-fluoro-9,10-dihydroacridin-9-one was reacted with sodium methanethiolate and the allyl group was cleaved from the resultant product to give 2-fluoro-10-(4-hydroxy-3,5-dimethoxybenzyl)-3-methylthio-9,10-dihydroacridin-9-one in 50% yield, m.p. >220°C (decomposes).

NMR ($CDCl_3$) 3.8(s, 6H), 5.5(s, 2H), 6.4(s, 2H), 7.0(d, 1H), 7.3(t, 1H), 7.4(d, 1H), 7.7(m, 1H), 8.1(d, 1H), 8.6(m, 1H).

## EXAMPLE 16

Using a similar procedure to that described in the first paragraph of Example 1, 3-fluoro-9,10-dihydroacridin-9-one was reacted with 4-allyloxy-3,5-dimethoxybenzyl chloride to give 10-(4-allyloxy-3,5-dimethoxybenzyl)-3-fluoro-9,10-dihydroacridin-9-one in 70% yield, m.p. 207-209°C (recrystallised from a mixture of hexane and dichloromethane).

The allyloxy group was cleaved using a similar procedure to that described in the second paragraph of Example 12. There was thus obtained 3-fluoro-10-(4-hydroxy-3,5-dimethoxybenzyl)-9,10-dihydroacridin-9-one in 35% yield, m.p. 210-213°C.

The 3-fluoro-9,10-dihydroacridin-9-one, used as a starting material, was obtained as follows:

A mixture of 2-chloro-4-fluorobenzoic acid (8.75g) and copper bronze (0.2g) was heated to 140°C for 3 hours. The excess of aniline was removed by steam distillation. The residue was filtered and the filtrate was acidified by the addition of concentrated aqueous hydrochloric acid (10ml). The solid was isolated and dried to give 2-anilino-4-fluorobenzoic acid (9.5g), m.p. 185-191°C.

A mixture of a portion (2g) of the product so obtained and polyphosphoric acid (25g) was heated to 140°C for 3 hours. The mixture was poured onto a mixture of ice and dilute aqueous ammonium hydroxide solution. The precipitate so obtained was washed with a saturated sodium bicarbonate solution and with water and then dried. There was thus obtained 3-fluoro-9,10-dihydroacridin-9-one (1.6g, 87%).

## EXAMPLE 17

Using a similar procedure to that described in Example 12, 10-(4-allyloxy-3,5-dimethoxybenzyl)-1-fluoro-9,10-dihydroacridin-9-one was reacted with allyl alcohol and potassium tert-butoxide by heating the reaction mixture to 85°C for 1 hour. There was thus obtained 1-allyloxy-10-(4-allyloxy-3,5-dimethoxybenzyl)-9,10-dihydroacridin-9-one in 82% yield.

The allyloxy groups were cleaved using a similar procedure to that described in the second paragraph of Example 12. There was thus obtained 1-hydroxy-10-(4-hydroxy-3,5-dimethoxybenzyl)-9,10-dihydroacridin-9-one in 27% yield, m.p. >210°C (decomposes).

NMR ($CD_3SOCD_3$) 3.6(s, 6H), 5.6(s, 2H), 6.4(s, 2H), 6.7(d, 1H), 7.0(d, 1H), 7.4(m, 1H), 7.7(m, 2H), 7.8(m, 1H), 8.4(m, 1H), 14.5(s, 1H).

The 10-(4-allyloxy-3,5-dimethoxybenzyl)-1-fluoro-9,10-dihydroacridin-9-one, used as a starting material, was obtained as follows:

Using a similar procedure to that described in the first paragraph of the portion of Example 12 which is concerned with preparation of starting materials, 1-fluoro-9,10-dihydroacridin-9-one was reacted with 4-allyloxy-3,5-dimethoxybenzyl chloride.

## EXAMPLE 18

The procedure described in the first paragraph of Example 6 was repeated except that the reaction was carried out at 0°C for 1 hour rather than at ambient temperature for 2 hours. There was thus obtained 10-(4-tert-butyldimethylsilyloxy-3,5-dimethoxybenzyl)-3-methylthio-9,10-dihydroacridin-9-one in 66% yield.

A mixture of a portion (0.14g) of the material so obtained, 3-chloroperoxybenzoic acid (0.15g) and dichloromethane (15ml) was stirred at ambient temperature for 2 hours. The mixture was basified by the addition of a saturated aqueous sodium bicarbonate solution. The organic phase was washed with water and with brine, dried ($MgSO_4$) and evaporated. The residue was purified by column chromatography using a 4:1 v/v mixture of dichloromethane and ethyl acetate as eluent. There was thus obtained 10-(4-tert-butyldimethylsilyloxy-3,5-dimethoxybenzyl)-3-methylsulphonyl-9,10-dihydroacridin-9-one (0.08g, 54%).

The procedure described in the second paragraph of Example 1 was employed to cleave the silyl group. There was thus obtained 10-(4-hydroxy-3,5-dimethoxybenzyl)-3-methylsulphonyl-9,10-dihydroacridin-9-one as a solid (0.005g, 8%).

NMR ($CDCl_3$): 3.0(s, 3H), 3.8(s, 6H), 5.5(s, 1H), 5.6(s, 2H), 6.4(s, 2H), 7.4(m, 1H), 7.5(d, 1H), 7.7(m, 2H), 8.1(s, 1H), 8.6(m, 1H), 8.8(d, 1H).

## EXAMPLE 19

A solution of potassium peroxymonosulphate (0.35g) in water (2ml) was added to a solution of 10-(4-allyloxy-3,5-dimethoxybenzyl)-3-(2-diethylaminoethylthio)-9,10-dihydroacridin-9-one (0.132g) in ethanol (5ml) and the mixture was stirred at ambient temperature for 2 hours. The mixture was evaporated and the residue was partitioned between ethyl acetate and water. The organic phase was washed with water and with brine, dried ($MgSO_4$) and evaporated. The residue was purified by column chromatography using a 10:3 v/v mixture of dichloromethane and ethyl acetate as eluent. There was thus obtained 10-(4-allyloxy-3,5-dimethoxybenzyl)-3-(2-diethylaminoethylsulphonyl)-9,10-dihydroacridin-9-one (0.08g).

The allyloxy group was cleaved using a similar procedure to that described in the second paragraph of Example 12. There was thus obtained 10-(4-hydroxy-3,5-dimethoxybenzyl)-3-(2-diethylaminoethylsulphonyl)-9,10-dihydroacridin-9-one (0.015g, 23%), m.p. 164-166°C (decomposes).

## EXAMPLE 20

10-(4-Allyloxy-3,5-dimethoxybenzyl)-3-(2-dimethylaminoethylthio)-9,10-dihydroacridin-9-one was oxidised with potassium peroxymonosulphate using the procedure described in Example 19 except that the chromatographic purification step was not required. The product was dissolved in acetonitrile and precipitated on the addition of a saturated solution of dimethylamine in acetonitrile (5ml). There was thus obtained 10-(4-allyloxy-3,5-dimethoxybenzyl)-3-(2-dimethylaminoethylsulphonyl)-9,10-dihydroacridin-9,10-dihydroacridin-9 -one in 94% yield, m.p. 149-151°C (recrystallised from a mixture of dichloromethane and ethanol).

The allyloxy group was cleaved using a similar procedure to that described in the second paragraph of Example 12. There was thus obtained 3-(2-dimethylaminoethylsulphonyl)-10-(4-hydroxy-3,5-dimethoxybenzyl)-9,10-dihydroacridin-9-one in 50% yield, m.p. 184-185°C (recrystallised from a mixture of dichloromethane and ethanol).

The 10-(4-allyloxy-3,5-dimethoxybenzyl)-3-(2-dimethylaminoethylthio)-9,10-dihydroacridin-9-one, used as a starting material, was obtained as follows:-

Sodium hydride (65% w/w dispersion in mineral oil, 0.7g) was added portionwise to a stirred suspension of 2-dimethylaminoethanethiol hydrochloride (1.21g) in 1-methylpyrrolidin-2-one (25ml) and the mixture was stirred at ambient temperature for 1 hour. A solution of 10-(4-allyloxy-3,5-dimethoxybenzyl)-3-fluoro-9,10-dihydroacridin-9-one (3.24g) in 1-methylpyrrolidin-2-one (20ml) was added and the mixture was stirred at ambient temperature for 16 hours. The mixture was partitioned between ethyl acetate and water. The organic phase was washed with water and with brine, dried ($MgSO_4$) and evaporated. The oil so obtained was triturated under a mixture of hexane and ethyl acetate. There was thus obtained 10-(4-allyloxy-3,5-dimethoxybenzyl)-3-(2-dimethylaminoethylthio)-9,10-dihydroacridin-9-one (3g, 77%) m.p. 152-153°C.

## EXAMPLE 21

A mixture of 3-(2-dimethylaminoethylthio)-10-(4-tert-butyldimethylsilyloxy-3,5-dimethoxybenzyl)-9,10-dihydroacridin-9-one (0.16g), 3-chloroperoxybenzoic acid (0.121g) and dichloromethane (20ml) was stirred at ambient temperature for 1 hour. The mixture was neutralised by the addition of a saturated aqueous sodium bicarbonate solution and the mixture was stirred at ambient temperature for 30 minutes. The organic phase was separated and combined with dichloromethane extracts (3 x 20ml) of the aqueous phase. The combined organic solutions were washed with water and with brine, dried ($MgSO_4$) and evaporated. The residue was purified by column chromatography using increasingly polar mixtures of dichloromethane and ethyl acetate as eluent. There was thus obtained 10-(4-tert-butyldimethylsilyloxy-3,5-dimethoxybenzyl)-3-vinylsulphonyl-9,10-dihydroacridin-9-one (0.05g, 33%).

The procedure described in the second paragraph of Example 1 was employed to cleave the silyl group. There was thus obtained 10-(4-hydroxy-3,5-dimethoxybenzyl)-3-vinylsulphonyl-9,10-dihydroacridin-9-one (0.026g, 63%), as a solid.

NMR ($CDCl_3$): 3.75(s, 6H), 5.5(s, 1H), 5.55(s, 2H), 6.0(d, 1H), 6.4(s, 2H), 6.5-6.65(m, 2H), 7.4(t, 1H), 7.5(d, 1H), 7.7(m, 2H), 8.05(s, 1H), 8.6(m, 1H), 8.7(d, 1H).

The 3-(2-dimethylaminoethylthio)-10-(4-tert-butyldimethylsilyloxy-3,5-dimethoxybenzyl)-9,10-dihydroacridin-9-one, used as a starting material, was obtained by the reaction of 3-fluoro-10-(4-tert-butyldimethylsilyloxy-3,5-dimethoxybenzyl)-9,10-dihydroacridin-9-one [obtained by the reaction of 3-fluoro-9,10-dihydroacridin-9-one with 4-tert-butyldimethylsilyloxy-3,5-dimethoxybenzyl chloride using a similar procedure to that described in the first paragraph of Example 1] with sodium 2-dimethylaminoethanethiol using a similar procedure to that described in Examples 6 and 7 except that the reaction mixture was stirred at ambient temperature for 15 hours.

## EXAMPLE 22

A solution of potassium peroxymonosulphate (2g) in water (15 ml) was added to a solution of 10-(4-(allyloxy-3,5-dimethoxybenzyl)-3-methylthio-9,10-dihydroacridin-9-one (1g) in ethanol (20ml) and the mixture was stirred at ambient temperature for 16 hours. Dichloromethane (50 ml) was added and the mixture was filtered. The filtrate was washed with water and with brine, dried ($MgSO_4$) and evaporated. The residue was purified by column chromatography using a 1:1 v/v mixture of hexane and ethyl acetate as eluent. There was thus

obtained 10-(4-allyloxy-3,5-dimethoxybenzyl)-3-methylsulphinyl-9,10-dihydroacridin-9-one (0.27g).

A solution of tetrakis(triphenylphosphine)palladium (0) [0.012g] in THF (0.25ml) was added to a mixture of a portion (0.1g) of the product so obtained, 2,2-dimethyl-1,3-dioxane-4,6-dione (0.09g) and DMF (2ml). The mixture was stirred at ambient temperature for 2 hours. A saturated aqueous sodium bicarbonate solution (1ml) was added and the mixture was partitioned between ethyl acetate and water. The organic phase was washed with water and with brine, dried ($MgSO_4$) and evaporated. The residue was triturated under ethyl acetate. There was thus obtained 10-(4-hydroxy-3,5-dimethoxybenzyl)-3-methylsulphinyl-9,10-dihydroacridin-9-one (0.04g), m.p. 221-223°C.

The 10-(4-allyloxy-3,5-dimethoxybenzyl)-3-methylthio-9,10-dihydroacridin-9-one, used as a starting material, was obtained as follows:-

A solution of 10-(4-allyloxy-3,5-dimethoxybenzyl)-3-fluoro-9,10-dihydroacridin-9-one (2g) in 1-methylpyrrolidin-2-one (20 ml) was stirred at ambient temperature and sodium methanethiolate (0.67g) was added portionwise. The mixture was stirred at ambient temperature for 1 hour. Water (150ml) was added and the mixture was stirred for 30 minutes. The precipitate was collected, washed with water and dried. There was thus obtained the required starting material (2.11g), m.p. 182-184°C.

## EXAMPLE 23

10-(4-hydroxy-3,5-dimethoxybenzyl)-3-(2-aminoethylthio)-9,10-dihydroacridin-9-one (0.077g) was oxidised with potassium peroxymonosulphate using a similar procedure to that described in Example 19. There was thus obtained 3-(2-aminoethylsulphonyl)-10-(4-hydroxy-3,5-dimethoxybenzyl)-9,10-dihydroacridin-9-one (0.01g, 14%) as a solid.

NMR ($CD_3SOCD_3$): 2.75(t, 2H), 3.55(t, 2H), 3.6(s, 6H), 5.75(s, 2H), 6.52(s, 2H), 7.4(m, 1H), 7.8(m, 3H), 8.2(d, 1H), 8.4(m, 1H), 8.6(d, 1H).

## EXAMPLE 24

Using a similar procedure to that described in Example 12, 10-(4-allyloxy-3,5-dimethoxybenzyl)-3-chloro-2-fluoro-9,10-dihydroacridin-9-one (0.057g) was reacted with sodium 2-dimethylaminoethanethiolate [prepared from sodium hydride and 2-dimethylaminoethiol in DMF (1ml)] to give 10-(4-allyloxy-3,5-dimethoxybenzyl)-3-(2-dimethylaminoethylthio)-2-fluoro-9,10-dihydroacridin-9-one (0.045g), 69%, m.p. 169-170°C.

The allyl group was cleaved from the resultant product using the procedure also described in Example 12 to give 3-(2-dimethylaminoethylthio)-2-fluoro-10-(4-hydroxy-3,5-dimethoxybenzyl)-9,10-dihydroacridin-9-one (0.01g, 27%), m.p. >170°C (decomposes).

NMR ($CD_3SOCD_3$): 2.1(s, 6H), 2.5(t, 2H), 3.1(t, 2H), 3.6(s, 6H), 5.7(s, 2H), 6.5(s, 2H), 7.4(m, 2H), 7.7(m, 2H), 7.9(d, 1H), 8.3(s, 1H), 8.35(m, 1H).

## EXAMPLE 25

A mixture of 10-(4-allyloxy-3,5-dimethoxybenzyl)-3-fluoro-9,10-dihydroacridin-9-one (0.31g), 2-aminoethanol (1ml), potassium tert-butoxide (0.18g) and DMF (5ml) was stirred and heated to 50°C for 1 hour. Water (10ml) was added and the mixture was extracted with ethyl acetate. The organic phase was washed with water and with brine, dried ($MgSO_4$) and evaporated. The residue was recrystallised from a mixture of hexane and ethyl acetate. There was thus obtained 10-(4-allyloxy-3,5-dimethoxybenzyl)-3-(2-aminoethoxy)-9,10-dihydroacridin-9-one (0.19g).

Using a similar procedure to that described in the second paragraph of Example 12 the allyl group was cleaved. There was thus obtained 3-(2-aminoethoxy-10-(4-hydroxy-3,5-dimethoxybenzyl-9,10-dihydroacridin-9-one (0.01g, 6%), m.p. 171°C (decomposes).

NMR ($CD_3SOCD_3$): 3.0(t, 2H), 3.6(s, 6H), 4.1(t, 2H), 5.6(s, 2H), 6.5(s, 2H), 7.0(m, 2H), 7.3(t, 1H), 7.7(m, 2H), 8.3(d, 1H), 8.4(m, 1H).

## EXAMPLE 26

A mixture of 10-(4-allyloxy-3,5-dimethoxybenzyl)-3-nitro-9,10-dihydroacridin-9-one (0.57g), stannous chloride (1.63g) and ethanol (20ml) was stirred and heated to 60°C. A solution of sodium borohydride (0.028g) in ethanol (1ml) was added dropwise and the resultant mixture was stirred at 60°C for 1 hour. Water (20ml) was added and the bulk of the ethanol was evaporated. The mixture was basified by the addition of 2N sodium hydroxide solution and extracted with dichloromethane (4 x 50ml). The combined extracts were dried ($MgSO_4$)

and evaporated. The residue was purified by recrystallisation from a mixture of hexane and dichloromethane. There was thus obtained 10-(4-allyloxy-3,5-dimethoxybenzyl)-3-amino-9,10-dihydroacridin-9-one (0.29g, 55%), m.p. 230°C.

Using a similar procedure to that described in the second paragraph of Example 12 the allyl group was cleaved. There was thus obtained 3-amino-10-(4-hydroxy-3,5-dimethoxybenzyl)-9,10-dihydroacridin-9-one in 36% yield, m.p. 199-201°C (decomposes).

NMR ($CD_3SOCD_3$) 3.6(s, 6H), 5.4(s, 2H), 6.46(s, 2H), 6.5(d, 1H), 6.6(m, 1H), 7.3(t, 1H), 7.4(d, 1H), 7.6(m, 1H), 8.0(d, 1H), 8.3(m, 1H).

The 10-(4-allyloxy-3,5-dimethoxybenzyl-3-nitro-9,10-dihydroacridin-9-one, used as a starting material, was obtained as follows:-

A mixture of 2-anilino-4-nitrobenzoic acid (20g; J. Chem. Soc., 1936, 1614) and polyphosphoric acid (300g) was heated to 140°C for 2 hours. The mixture was poured onto a mixture of water and ice and the resultant precipitate was collected. There was thus obtained 3-nitro-9,10-dihydroacridin-9-one (11g).

A mixture of a portion (9.5g) of the product so obtained, 4-allyloxy-3,5-dimethoxybenzyl chloride (12.5g), 2-butanone (45ml), 50% w/v aqueous sodium hydroxide solution (45ml) and benzyltriethylammonium chloride (0.5g) was stirred rapidly and heated to reflux for 2.25 hours. The mixture was cooled to ambient temperature and water (45ml) was added. The mixture was extracted with dichloromethane (3 x 40 ml). The combined organic extracts were washed with water and with brine, dried ($MgSO_4$) and evaporated. The residue was purified by column chromatography using a 99:1 v/v mixture of dichloromethane and methanol as eluent. There was thus obtained the required starting material (3.1g, 18%), m.p. 224-225°C (recrystallised from a mixture of hexane and dichloromethane).

**EXAMPLE 27**

A mixture of 10-(4-allyloxy-3,5-dimethoxybenzyl)-3-fluoro-9,10-dihydroacridin-9-one (1g), piperazine (2.05g) and 1-methylpyrtolidin-2-one (20ml) was stirred and heated to 140°C for 20 hours. The mixture was cooled to ambient temperature and partitioned between ethyl acetate and water. The organic phase was washed with water and with brine, dried ($MgSO_4$ ) and evaporated. The residue was purified by column chromatography using an 18:1:1 v/v mixture of dichloromethane, methanol and triethylamine as eluent. There was thus obtained 10-(4-allyloxy-3,5-dimethoxybenzyl)-3-piperazinyl-9,10-dihydroacridin-9-one (0.98g, 85%), m.p. 185-186°C.

A solution of tetrakis(triphenylphosphine)palladium(0) [0.034g] in THF (0.5ml) was added to a mixture of a portion (0.48g) of the product so obtained, Meldrum's acid (0.29g) and DMF (5ml). The mixture was stirred at ambient temperature for 1 hour. The mixture was partitioned between dichloromethane and a saturated aqueous sodium bicarbonate solution. The organic phase was washed with water, dried ($MgSO_4$) and evaporated. The residue was purified by column chromatography using an 18:1:1 v/v mixture of dichloromethane, methanol and triethylamine as eluent. There was thus obtained 10-(4-hydroxy-3,5-dimethoxybenzyl)-3-piperazinyl-9,10-dihydroacridin-9-one (0.095g, 22%), m.p. 205-207°C.

NMR ($CDCl_3$): 2.9(t, 4H), 3.2(t, 4H), 3.7(s, 6H), 5,5(s, 2H), 6.5(s, 2H), 6.65(s, 1H), 6.95(m, 1H), 7.25(t, 1H), 7.5(d, 1H), 7.65(m, 1H), 8.3(d, 1H), 8.4(m, 1H).

Elemental Analysis: Found C,68.8; H,6.1; N,9.3;

$C_{26}H_{27}N_3O_4$ $0.25H_2O$ requires C,68.6; H,6.2; N,9.2%.

**EXAMPLE 28**

Using a similar procedure to that described in Example 27, 10-(4-allyloxy-3,5-dimethoxybenzyl)-3-fluoro-9,10-dihydroacridin-9-one was reacted with morpholine and the allyl group was cleaved from the resultant product to give 10-(4-hydroxy-3,5-dimethoxybenzyl)-3-morpholino-9,10-dihydroacridin-9-one in 7.5% yield, m.p. 207-208°C.

NMR ($CDCl_3$ + $CD_3SOCD_3$): 3.28(t, 4H), 3.75(s, 6H), 3.8(t, 4H), 5.46(s, 2H), 6.46(s, 2H), 6.62(s, 1H), 6.9(m, 1H), 7.3(t, 1H), 7.42(d, 1H), 7.65(m, 1H), 8.4(d, 1H), 8.55(m, 1H).

Elemental Analysis: Found C,69.3; H,5.9; N,6.1;

$C_{26}H_{26}N_2O_5$ $0.25H_2O$ requires C,69.2; H,5.9; N,6.2%.

**EXAMPLE 29**

(Trimethylsilyl)acetylene (0.1ml) and bis(triphenylphosphine)palladium(II) chloride (0.023g) were added in turn to a mixture of 3-bromo-10-(4-tert-butyldimethylsilyloxy-3,5-dimethoxybenzyl)-9,10-dihydroacridin-9-one

(0.364g), cuprous iodide (0.013g), triethylamine (0.37ml) and THF (10ml). The mixture was stirred at ambient temperature for 24 hours. The mixture was partitioned between ethyl acetate and water. The organic phase was washed with water and with brine, dried ($MgSO_4$) and evaporated. The residue was purified by column chromatography using a 4:1 v/v mixture of hexane and ethyl acetate as eluent. There was thus obtained 10-(4-tert-butyldimethylsilyloxy-3,5-dimethoxybenzyl)-3-trimethylsilylethynyl-9,10-dihydroacridin-9-one (0.13g, 35%).

The procedure described in the second paragraph of Example 1 was employed to cleave the silyl groups. There was thus obtained 3-ethynyl-10-(4-hydroxy-3,5-dimethoxybenzyl)-9,10-dihydroacridin-9-one (0.03g, 61%); m.p. 157-159°C (recrystallised from a mixture of hexane and chloroform).

CHEMICAL FORMULAE

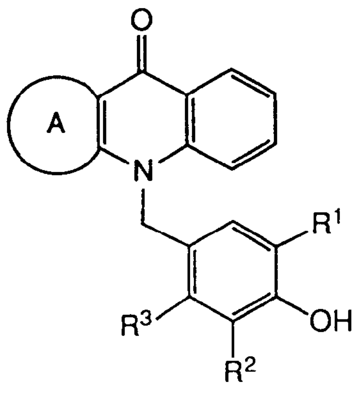

I

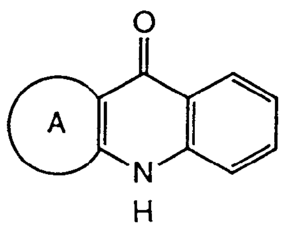

II

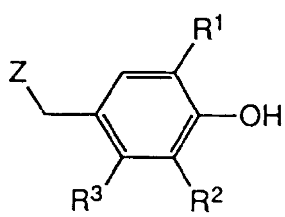

III

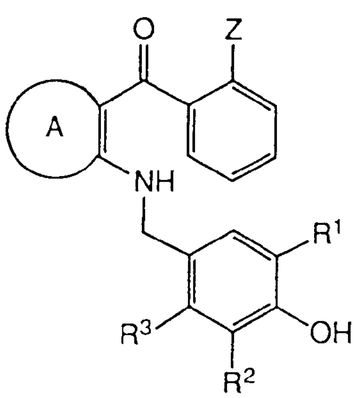

IV

## Claims

1. A tricyclic heterocyclic derivative of the formula I

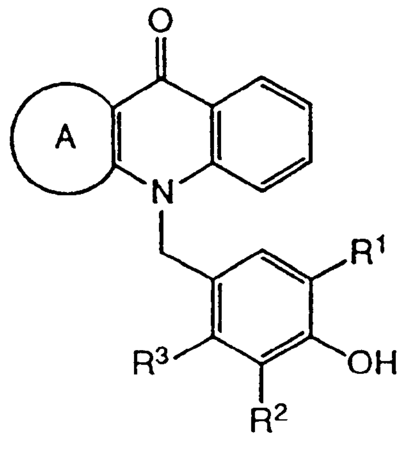

I

wherein A together with the adjacent vinylene group of the 4-oxo-1,4-dihydropyrid-1-yl ring completes a benzene or pyridine ring, which may optionally bear one, two or three substituents selected from halogeno, hydroxy, amino, (1-4C)alkyl, (2-4C)alkenyl, (2-C)alkynyl, (1-4C)alkoxy, (1-4C)alkylamino, di-[(1-4C)alkyl-]amino, piperidino, morpholino, piperazin-1-yl, 4-[(1-4C)alkyl]piperazin-1-yl, (1-6C)alkylthio; (1-6C)alkylsulphinyl, (1-6C)alkylsulphonyl, (2-4C)alkenylthio, (2-4C)alkenylsulphinyl, (2-4C)alkenylsulphonyl, (1-4C)alkylenedioxy, hydroxy-(1-4C)alkyl, hydroxy-(2-4C)alkylthio, hydroxy-(2-4C)alkylsulphinyl and hydroxy-(2-4C)alkylsulphonyl, from a group of the formula:-

$$-X^1-(CH_2)_m-N(R^4)-R^5$$

wherein $X^1$ is oxy, thio, sulphinyl; sulphonyl or imino, m is an integer from 2 to 4, and $R^4$ and $R^5$, which may be the same or different, each is hydrogen or (1-4C)alkyl, or $R^4$ and $R^5$ together with the nitrogen atom to which each is attached define a piperidino, morpholino, piperazin-1-yl o- 4-[(1-4C)alkyl]piperazin-1-yl ring, or $X^1$ is a direct link, m is an integer from 1 to 4 and $R^4$ and $R^5$ have any of the meanings defined above, and from phenyl-(1-4C)alkyl, phenyl-(1-4C)alkoxy, phenyl-(1-4C)alkylthio, phenyl-(1-4C)alkylsulphinyl and phenyl-(1-4C)alkylsulphonyl, and wherein any of said substituents comprising a phenyl group may optionally bear a halogeno, (1-4C)alkyl or (1-4C)alkoxy substituent;

$R^1$ and $R^2$, which may be the same or different, each is (1-4C)alkyl or (1-4C)alkoxy;

and $R^3$ is hydrogen, (1-4C)alkyl or (1-4C)alkoxy;

or a pharmaceutically-acceptable salt or in vivo hydrolysable ester thereof.

2. A tricyclic heterocyclic derivative of the formula I as claimed in claim 1 wherein A together with the adjacent vinylene group of the 4-oxo-1,4-dihydroquinolin-1-yl ring completes a benzene or pyridine ring, which may optionally bear one, two or three substituents selected from fluoro, chloro; bromo, hydroxy, amino, methyl, ethyl, vinyl, ethynyl, methoxy, ethoxy; methylamino, ethylamino, dimethylamino, piperidino, morpholino, piperazin-1-yl, 4-methylpiperazin-1-yl, methylthio, ethylthio, propylthio, isopropylthio, isobutylthio, isopentylthio, methylsulphinyl, ethylsulphinyl, methylsulphonyl, ethylsulphonyl, vinylthio, vinylsulphinyl, vinylsulphonyl, methylenedioxy, 2-hydroxyethyl, 2-hydroxyethylthio; 2-hydroxyethylsulphinyl and 2-hydroxyethylsulphonyl,

from a group of the formula:-

$$-X^1-(CH_2)_m-N(R^4)-R^5.$$

wherein $X^1$ is oxy, thio, sulphinyl, sulphonyl or imino, m is 2 or 3, and $R^4$ and $R^5$, which may be the same or different; each is hydrogen, methyl or ethyl, or $R^4$ and $R^5$ together with the nitrogen atom to which each is attached define a piperidino, morpholino, piperazin-1-yl or 4-methylpiperazin-1-yl ring, or $X^1$ is a direct link, m is an integer from 1 to 3 and $R^4$ and $R^5$ have any of the meanings defined above, and from benzyl, phenethyl, benzyloxy and benzylthio and wherein any of said substituents comprising a phenyl group may optionally bear a substituent selected from fluoro, chloro, methyl and methoxy;

$R^1$ and $R^2$, which may be the same or different, each is methyl, ethyl, methoxy or ethoxy;

and $R^3$ is hydrogen, methyl, ethyl, methoxy or ethoxy;

or a pharmaceutically-acceptable salt or in vivo hydrolysable ester thereof.

3. A tricyclic heterocyclic derivative of the formula I as claimed in claim 1 wherein A together with the adjacent vinylene group of the 4-oxo-1,4-dihydroquinolin-1-yl ring completes a benzene or pyridine ring; the tricyclic heterocyclic radical so defined may optionally bear one or two substituents selected from fluoro, chloro, bromo, hydroxy, amino, methyl, ethyl, methoxy, ethoxy, methylamino; ethylamino, dimethylamino, piperidino, morpholino, piperazin-1-yl, 4-methylpiperazin-1-yl, methylthio, ethylthio; propylthio, isopropylthio, isobutylthio, isopentylthio, methylsulphinyl, ethylsulphinyl, methylsulphonyl, ethylsulphonyl and methylenedioxy, from a group of the formula:-

$$-X^1-(CH_2)_m-N(R^4)-R^5$$

wherein $X^1$ is oxy, thio or imino, m is 2, and $R^4$ and $R^5$, which may be the same or different, each is hydrogen, methyl or ethyl, or $R^4$ and $R^5$ together with the nitrogen atom to which each is attached define a piperidino, morpholino, piperazin-1-yl or 4-methylpiperazin-1-yl ring, and from benzyl, phenethyl, benzyloxy and benzylthio and wherein any of said substituents comprising a phenyl group may optionally bear a substituent selected from fluoro, chloro, methyl and methoxy;

$R^1$ and $R^2$, which may be the same or different, each is methyl, ethyl, methoxy or ethoxy;
and $R^3$ is hydrogen, methyl, ethyl, methoxy or ethoxy;
or a pharmaceutically-acceptable salt or in vivo hydrolysable ester thereof.

4. A tricyclic heterocyclic derivative of the formula I as claimed in claim 1 wherein the tricyclic heterocyclic radical defined by the 4-oxo-1,4-dihydroquinolin-1-yl ring and A is 9-oxo-9,10-dihydroacridin-10-yl or 5-oxo-5,10-dihydrobenzo[b][1,8]naphthyridin-10-yl, which may optionally bear one or two substituents selected from fluoro, chloro, bromo, hydroxy, amino, ethynyl, methoxy, ethoxy, piperidino, morpholino, piperazin-1-yl, 4-methylpiperazin-1-yl, methylthio; ethylthio, isopentylthio, methylsulphinyl, ethylsulphinyl, methylsulphonyl, ethylsulphonyl, vinylthio, vinylsulphinyl, vinylsulphonyl, methylenedioxy, 2-hydroxyethylthio, 2-aminoethoxy, 2-dimethylaminoethoxy, 2-diethylaminoethoxy, 2-aminoethylthio, 2-dimethylaminoethylthio, 2-diethylaminoethylthio, 2-aminoethylsulphinyl, 2-dimethylaminoethylsulphinyl, 2-diethylaminoethylsulphinyl, 2-aminoethylsulphonyl, 2-dimethylaminoethylsulphonyl, 2-diethylaminoethylsulphonyl, 2-dimethylaminoethylamino and benzylthio;

$R^1$ is methoxy;
$R^2$ is methoxy; and
$R^3$ is hydrogen;
or a pharmaceutically-acceptable salt or in vivo hydrolysable ester thereof.

5. A tricyclic heterocyclic derivative of the formula I as claimed in claim 1

wherein the tricyclic heterocyclic radical defined by the 4-oxo-1,4-dihydroquinolin-1-yl ring and A is 9-oxo-9,10-dihydroacridin-10-yl or 5-oxo-5,10-dihydrobenzo[b][1,8]naphthyridin-10-yl, which may optionally bear one substituent selected from chloro, methylthio, methylenedioxy and 2-dimethylaminoethylthio;

$R^1$ is methoxy;
$R^2$ is methoxy; and
$R^3$ is hydrogen;
or a pharmaceutically-acceptable salt thereof.

6. A tricyclic heterocyclic derivative of the formula I as claimed in claim 1 wherein the tricyclic heterocyclic radical defined by the 4-oxo-1,4-dihydroquinolin-1-yl ring and A is 9-oxo-9,10-dihydroacridin-10-yl which bears one or two substituents selected from fluoro, chloro, hydroxy, methoxy, methylthio, methylsulphinyl, methylsulphonyl, methylenedioxy, 2-aminoethoxy, 2-aminoethylthio, 2-dimethylaminoethylthio, 2-aminoethylsulphonyl and 2-dimethylaminoethylsulphonyl;

$R^1$ is methoxy;
$R^2$ is methoxy; and
$R^3$ is hydrogen;
or a pharmaceutically-acceptable salt thereof.

7. A tricyclic heterocyclic derivative of the formula I, or a pharmaceutically-acceptable salt thereof, as claimed in claim 1 selected from:-

3-chloro-10-(4-hydroxy-3,5-dimethoxybenzyl)-9,10-dihydroacridin-9-one, 10-(4-hydroxy-3,5-dimethoxybenzyl)-2,3-methylenedioxy-9,10-dihydroacridin-9-one,

10-(4-hydroxy-3,5-dimethoxybenzyl)-3-methylthio-9,10-dihydroacridin-9-one;

3-(2-dimethylaminoethylthio)-10-(4-hydroxy-3,5-dimethoxybenzyl)-9,10-dihydroacridin-9-one,

10-(4-hydroxy-3,5-dimethoxybenzyl)-5,10-dihydrobenzo[b][1,8]naphthyridin-5-one,
3-fluoro-10-(4-hydroxy-3,5-dimethoxybenzyl)-9,10-dihydroacridin-9-one,
3-chloro-2-fluoro-10-(4-hydroxy-3,5-dimethoxybenzyl)-9,10-dihydroacridin-9-one;
3-hydroxy-10-(4-hydroxy-3,5-dimethoxybenzyl)-9,10-dihydroacridin-9-one,
10-(4-hydroxy-3,5-dimethoxybenzyl)-3-(piperazin-1-yl)-9,10-dihydroacridin-9-one,
3-(2-dimethylaminoethylsulphonyl)-10-(4-hydroxy-3,5-dimethoxybenzyl)-9,10-dihydroacridin-9-one,
3-(2-aminoethoxy)-10-(4-hydroxy-3,5-dimethoxybenzyl)-9,10-dihydroacridin-9-one and
3-(2-aminoethylsulphonyl)-10-(4-hydroxy-3,5-dimethoxybenzyl)-9,10-dihydroacridin-9-one.

8. A process for the preparation of a tricyclic heterocyclic derivative of the formula I, or a pharmaceutically-acceptable salt or in vivo hydrolysable ester thereof, as claimed in claim 1 which comprises:-

(a) the coupling of a compound of the formula II

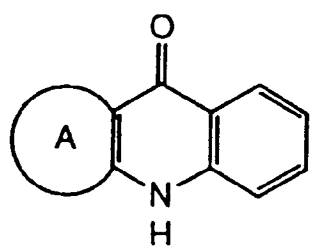

II

with a compound of the formula III

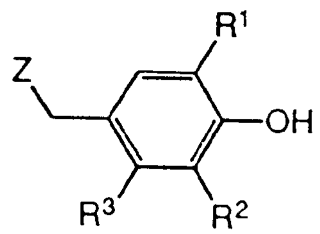

III

wherein Z is a displaceable group;

provided that the phenolic hydroxy group in the compound of the formula III and any amino, alkylamino, piperazinyl or other hydroxy group in the compounds of the formulae II and III may be protected by a conventional protecting group or alternatively any such group need not be protected, whereafter any such protecting group is removed by conventional means;

(b) the cyclisation of a compound of the formula IV

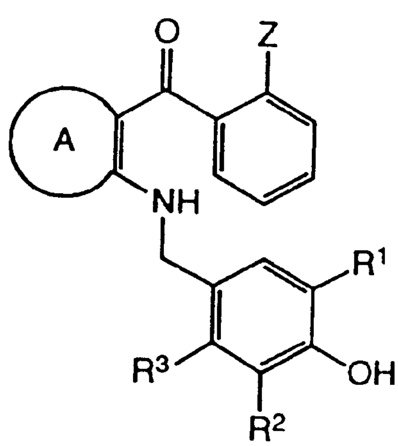

IV

wherein Z is a displaceable group as defined hereinbefore;

(c) for the production of a compound of the formula I wherein the tricyclic heterocyclic radical bears a hydroxy or substituted hydroxy substituent, an amino or substituted amino substituent or a substituted mercapto substituent, the displacement reaction of a compound of the formula I which bears a displaceable substituent Z as defined hereinbefore with an appropriate alcohol; amine or thiol;
(d) for the production of a compound of the formula I wherein the tricyclic heterocyclic radical bears a substituent comprising a sulphinyl or sulphonyl group; the oxidation of a compound of the formula I wherein the tricyclic heterocyclic radical bears an appropriate substituent comprising a thio group;
(e) for the production of a compound of the formula I wherein the tricyclic heterocyclic radical bears a substituent of the formula:-

$$-X^1-(CH_2)_m-N(R^4)-R^5$$

and $X^1$ is oxy, thio or imino, the alkylation of a compound of the formula I wherein the tricyclic heterocyclic radical bears a substituent of the formula $-X^1-H$ with a compound of the formula:- $Z-(CH_2)_m-N(R^4)-R^5$ wherein Z is a displaceable group; or
(f) for the production of a compound of the formula I wherein the tricyclic heterocyclic radical bears an amino substituent, the reduction of a compound of the formula I wherein the tricyclic heterocyclic radical bears a nitro substituent;

and when a pharmaceutically-acceptable salt of a novel compound of the formula I is required, it may be obtained by reaction of said compound with a suitable acid or base using a conventional procedure;

and when an in vivo hydrolysable ester of a novel compound of the formula I is required, it may be obtained by reaction of said compound with a suitable esterifying reagent using a conventional procedure.

9. A pharmaceutical composition which comprises a tricyclic heterocyclic derivative of the formula I, or a pharmaceutically-acceptable salt or in vivo hydrolysable ester thereof, as claimed in any one of claims 1 to 7 in association with a pharmaceutically-acceptable diluent or carrier.

10. The use of a tricyclic heterocyclic derivative of the formula I, or a pharmaceutically-acceptable salt or in vivo hydrolysable ester thereof, as claimed in any one of claims 1 to 7 in the manufacture of a medicament for use in the production of an anti-cancer effect in the human or animal body.

**Claims for the following Contracting States: GR-ES**

1. A process for the preparation of a tricyclic heterocyclic derivative of the formula I

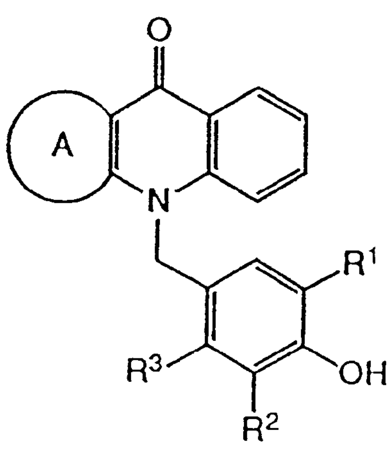

I

wherein A together with the adjacent vinylene group of the 4-oxo-1,4-dihydropyrid-1-yl ring completes a benzene or pyridine ring, which may optionally bear one, two or three substituents selected from halogeno, hydroxy, amino, (1-4C)alkyl; (2-4C)alkenyl, (2-C)alkynyl, (1-4C)alkoxy, (1-4C)alkylamino, di-[(1-4C)alkyl-]amino, piperidino, morpholino, piperazin-1-yl, 4-[(1-4C)alkyl]piperazin-1-yl, (1-6C)alkylthio, (1-6C)alkylsulphinyl, (1-6C)alkylsulphonyl, (2-4C)alkenylthio, (2-4C)alkenylsulphinyl, (2-4C)alkenylsulphonyl, (1-4C) alkylenedioxy, hydroxy-(1-4C)alkyl, hydioxy-(2-4C)alkylthio, hydroxy-(2-4C)alkylsulphinyl and hydroxy-(2-4C)alkylsulphonyl, from a group of the formula:-

$$-X^1-(CH_2)_m-N(R^4)-R^5$$

wherein $X^1$ is oxy, thio, sulphinyl, sulphonyl or imino, m is an integer from 2 to 4, and $R^4$ and $R^5$, which may be the same or different, each is hydrogen or (1-4C)alkyl, or $R^4$ and $R^5$ together with the nitrogen atom to which each is attached define a piperidino, morpholino, piperazin-1-yl or 4-[(1-4C)alkyl]piperazin-1-yl ring, or $X^1$ is a direct link, m is an integer from 1 to 4 and $R^4$ and $R^5$ have any of the meanings defined above, and from phenyl-(1-4C)alkyl, phenyl-(1-4C)alkoxy, phenyl-(1-4C)alkylthio, phenyl-(1-4C)alkylsulphinyl and phenyl-(1-4C)alkylsulphonyl, and wherein any of said substituents comprising a phenyl group may optionally bear a halogeno, (1-4C)alkyl or (1-4C)alkoxy substituent;

$R^1$ and $R^2$, which may be the same or different, each is (1-4C)alkyl or (1-4C)alkoxy;

and $R^3$ is hydrogen, (1-4C)alkyl or (1-4C)alkoxy;

or a pharmaceutically-acceptable salt or in vivo hydrolysable ester thereof;

characterised by

(a) the coupling of a compound of the formula II

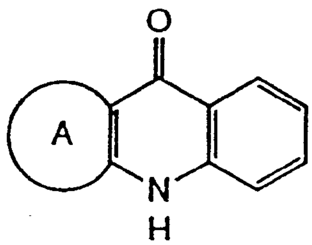

II

with a compound of the formula III

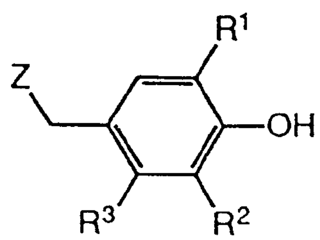

III

wherein Z is a displaceable group;

provided that the phenolic hydroxy group in the compound of the formula III and any amino, alkylamino, piperazinyl or other hydroxy group in the compounds of the formulae II and III may be protected by a conventional protecting group or alternatively any such group need not be protected, whereafter any such protecting group is removed by conventional means;

(b) the cyclisation of a compound of the formula IV

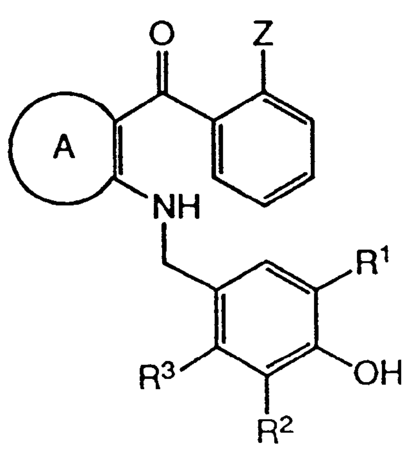

IV

wherein Z is a displaceable group as defined hereinbefore;

(c) for the production of a compound of the formula I wherein the tricyclic heterocyclic radical bears a hydroxy or substituted hydroxy substituent, an amino or substituted amino substituent or a substituted mercapto substituent, the displacement reaction of a compound of the formula I which bears a displaceable substituent Z as defined hereinbefore with an appropriate alcohol, amine or thiol;
(d) for the production of a compound of the formula I wherein the tricyclic heterocyclic radical bears a substituent comprising a sulphinyl or sulphonyl group, the oxidation of a compound of the formula I wherein the tricyclic heterocyclic radical bears an appropriate substituent comprising a thio group;
(e) for the production of a compound of the formula I wherein the tricyclic heterocyclic radical bears a substituent of the formula:-

$$-X^1-(CH_2)_m-N(R^4)-R^5$$

and $X^1$ is oxy, thio or imino, the alkylation of a compound of the formula I wherein the tricyclic heterocyclic radical bears a substituent of the formula $-X^1-H$ with a compound of the formula:- $Z-(CH_2)_m-N(R^4)-R^5$ wherein Z is a displaceable group; or
(f) for the production of a compound of the formula I wherein the tricyclic heterocyclic radical bears an amino substituent, the reduction of a compound of the formula I wherein the tricyclic heterocyclic radical bears a nitro substituent;

and when a pharmaceutically-acceptable salt of a novel compound of the formula I is required, it may be obtained by reaction of said compound with a suitable acid or base using a conventional procedure;

and when an in vivo hydrolysable ester of a novel compound of the formula I is required, it may be obtained by reaction of said compound with a suitable esterifying reagent using a conventional procedure.

**2.** A process as claimed in claim 1 for the preparation of a tricyclic heterocyclic derivative of the formula I wherein A together with the adjacent vinylene group of the 4-oxo-1,4-dihydroquinolin-1-yl ring completes a benzene or pyridine ring, which may optionally bear one, two or three substituents selected from fluoro, chloro, bromo, hydroxy, amino, methyl, ethyl, vinyl, ethynyl, methoxy, ethoxy, methylamino, ethylamino, dimethylamino; piperidino, morpholino, piperazin-1-yl, 4-methylpiperazin-1-yl, methylthio, ethylthio, propylthio, isopropylthio, isobutylthio, isopentylthio, methylsulphinyl, ethylsulphinyl, methylsulphonyl, ethylsulphonyl, vinylthio, vinylsulphinyl, vinylsulphonyl, methylenedioxy, 2-hydroxyethyl, 2-hydroxyethylthio, 2-hydroxyethylsulphinyl and 2-hydroxyethylsulphonyl, from a group of the formula:-

$$-X^1-(CH_2)_m-N(R^4)-R^5.$$

wherein $X^1$ is oxy, thio, sulphinyl, sulphonyl or imino, m is 2 or 3, and $R^4$ and $R^5$, which may be the same or different, each is hydrogen, methyl or ethyl, or $R^4$ and $R^5$ together with the nitrogen atom to which each is attached define a piperidino, morpholino, piperazin-1-yl or 4-methylpiperazin-1-yl ring, or $X^1$ is a direct link, m is an integer from 1 to 3 and $R^4$ and $R^5$ have any of the meanings defined above, and from benzyl, phenethyl, benzyloxy and benzylthio and wherein any of said substituents comprising a phenyl group may optionally bear a substituent selected from fluoro, chloro, methyl and methoxy;

$R^1$ and $R^2$, which may be the same or different, each is methyl, ethyl, methoxy or ethoxy;
and $R^3$ is hydrogen, methyl, ethyl, methoxy or ethoxy;
or a pharmaceutically-acceptable salt or in vivo hydrolysable ester thereof.

**3.** A process as claimed in claim 1 for the preparation of a tricyclic heterocyclic derivative of the formula I wherein A together with the adjacent vinylene group of the 4-oxo-1,4-dihydroquinolin-1-yl ring completes a benzene or pyridine ring; the tricyclic heterocyclic radical so defined may optionally bear one or two substituents selected from fluoro, chloro, bromo, hydroxy; amino, methyl, ethyl, methoxy, ethoxy, methylamino, ethylamino, dimethylamino, piperidino, morpholino, piperazin-1-yl, 4-methylpiperazin-1-yl, methylthio, ethylthio, propylthio, isopropylthio, isobutylthio, isopentylthio, methylsulphinyl, ethylsulphinyl, methylsulphonyl, ethylsulphonyl and methylenedioxy; from a group of the formula:-

$$-X^1-(CH_2)_m-N(R^4)-R^5$$

wherein $X^1$ is oxy, thio or imino, m is 2, and $R^4$ and $R^5$, which may be the same or different, each is hydrogen, methyl or ethyl, or $R^4$ and $R^5$ together with the nitrogen atom to which each is attached define a piperidino, morpholino, piperazin-1-yl or 4-methylpiperazin-1-yl ring, and from benzyl, phenethyl, benzyloxy and benzylthio and wherein any of said substituents comprising a phenyl group may optionally bear a substituent selected from fluoro, chloro, methyl and methoxy;

$R^1$ and $R^2$, which may be the same or different, each is methyl, ethyl, methoxy or ethoxy;
and $R^3$ is hydrogen, methyl, ethyl, methoxy or ethoxy;
or a pharmaceutically-acceptable salt or in vivo hydrolysable ester thereof.

4. A process as claimed in claim 1 for the preparation of a tricyclic heterocyclic derivative of the formula I wherein the tricyclic heterocyclic radical defined by the 4-oxo-1,4-dihydroquinolin-1-yl ring and A is 9-oxo-9,10-dihydroacridin-10-yl or 5-oxo-5,10-dihydrobenzo[b][1;8]naphthyridin-10-yl, which may optionally bear one or two substituents selected from fluoro, chloro, bromo, hydroxy, amino, ethynyl, methoxy, ethoxy, piperidino, morpholino, piperazin-1-yl, 4-methylpiperazin-1-yl, methylthio, , ethylthio, isopentylthio, methylsulphinyl, ethylsulphinyl, methylsulphonyl, ethylsulphonyl, vinylthio, vinylsulphinyl, vinylsulphonyl, methylenedioxy, 2-hydroxyethylthio; 2-aminoethoxy, 2-dimethylaminoethoxy, 2-diethylaminoethoxy, 2-aminoethylthio, 2-dimethylaminoethylthio, 2-diethylaminoethylthio, 2-aminoethylsulphinyl, 2-dimethylaminoethylsulphinyl, 2-diethylaminoethylsulphinyl, 2-aminoethylsulphonyl, 2-dimethylaminoethylsulphonyl; 2-diethylaminoethylsulphonyl, 2-dimethylaminoethylamino and benzylthio;
   $R^1$ is methoxy;
   $R^2$ is methoxy; and
   $R^3$ is hydrogen;
   or a pharmaceutically-acceptable salt or in vivo hydrolysable ester thereof.

5. A process as claimed in claim 1 for the preparation of a tricyclic heterocyclic derivative of the formula I wherein the tricyclic heterocyclic radical defined by the 4-oxo-1,4-dihydroquinolin-1-yl ring and A is 9-oxo-9,10-dihydroacridin-10-yl or 5-oxo-5,10-dihydrobenzo[b][1,8]naphthyridin-10-yl, which may optionally bear one substituent selected from chloro, methylthio, methylenedioxy and 2-dimethylaminoethylthio;
   $R^1$ is methoxy;
   $R^2$ is methoxy; and
   $R^3$ is hydrogen;
   or a pharmaceutically-acceptable salt thereof.

6. A process as claimed in claim 1 for the preparation of a tricyclic heterocyclic derivative of the formula I wherein the tricyclic heterocyclic radical defined by the 4-oxo-1,4-dihydroquinolin-1-yl ring and A is 9-oxo-9,10-dihydroacridin-10-yl which bears one or two substituents selected from fluoro, chloro, hydroxy, methoxy, methylthio, methylsulphinyl, methylsulphonyl, methylenedioxy, 2-aminoethoxy, 2-aminoethylthio, 2-dimethylaminoethylthio, 2-aminoethylsulphonyl and 2-dimethylaminoethylsulphonyl;
   $R^1$ is methoxy;
   $R^2$ is methoxy; and
   $R^3$ is hydrogen;
   or a pharmaceutically-acceptable salt thereof.

7. A process as claimed in claim 1 for the preparation of a tricyclic heterocyclic derivative of the formula I, or a pharmaceutically-acceptable salt thereof, selected from:-
   3-chloro-10-(4-hydroxy-3,5-dimethoxybenzyl)-9,10-dihydroacridin-9-one,
   10-(4-hydroxy-3;5-dimethoxybenzyl)-2,3-methylenedioxy-9,10-dihydroacridin-9-one,
   10-(4-hydroxy-3,5-dimethoxybenzyl)-3-methylthio-9,10-dihydroacridin-9-one,
   3-(2-dimethylaminoethylthio)-10-(4-hydroxy-3,5-dimethoxybenzyl)-9,10-dihydroacridin-9-one,
   10-(4-hydroxy-3,5-dimethoxybenzyl)-5,10-dihydrobenzo[b][1,8]naphthyridin-5-one,
   3-fluoro-10-(4-hydroxy-3,5-dimethoxybenzyl)-9,10-dihydroacridin-9-one,
   3-chloro-2-fluoro-10-(4-hydroxy-3,5-dimethoxybenzyl)-9,10-dihydroacridin-9-one,
   3-hydroxy-10-(4-hydroxy-3,5-dimethoxybenzyl)-9,10-dihydroacridin-9-one,
   10-(4-hydroxy-3,5-dimethoxybenzyl)-3-(piperazin-1-yl)-9,10-dihydroacridin-9-one,
   3-(2-dimethylaminoethylsulphonyl)-10-(4-hydroxy-3,5-dimethoxybenzyl)-9,10-dihydroacridin-9-one,
   3-(2-aminoethoxy)-10-(4-hydroxy-3,5-dimethoxybenzyl)-9,10-dihydroacridin-9-one and
   3-(2-aminoethylsulphonyl)-10-(4-hydroxy-3,5-dimethoxybenzyl)-9,10-dihydroacridin-9-one.

8. A process for the preparation of a pharmaceutical composition which comprises bringing into admixture a tricyclic heterocyclic derivative of the formula I, or a pharmaceutically-acceptable salt or in vivo hydrolysable ester thereof, as defined in any one of claims 1 to 7 and a pharmaceutically-acceptable diluent or carrier.

9. The use of a tricyclic heterocyclic derivative of the formula I, or a pharmaceutically-acceptable salt or in vivo hydrolysable ester thereof, as defined in any one of claims 1 to 7 in the manufacture of a medicament for use in the production of an anti-cancer effect in the human or animal body.

European Patent Office

# EUROPEAN SEARCH REPORT

Application Number

EP 91307341.7

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| A | DE - A1 - 2 759 468 (STERLING DRUG) * Claims 1,5; page 4, lines 9-14 * -- | 1,8,9 | C 07 D 219/06<br>C 07 D 219/08<br>C 07 D 471/04<br>C 07 D 491/04<br>A 61 K 31/435 |
| A | GB - A - 1 228 196 (INSTITUT FARMACEUT.) * Claims 1,3,4; page 1, lines 29-32 * ---- | 1,8,9 | |
| | | | TECHNICAL FIELDS SEARCHED (Int. Cl.5) |
| | | | C 07 D 219/00<br>C 07 D 471/00<br>C 07 D 491/00 |

The present search report has been drawn up for all claims

| Place of search | Date of completion of the search | Examiner |
|---|---|---|
| VIENNA | 08-11-1991 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)